# EUROPEAN PATENT APPLICATION

(11) **EP 2 589 385 A1**
(43) Date of publication of application: **08.05.2013**
(21) Application number: 11382337.1
(22) Date of filing: 03.11.2011
(51) Int. Cl.: A61K 31/4439, A61K 31/7068, A61K 45/06, A61P 35/00

(54) **TRIPLE COMBINATION FOR THE TREATMENT OF CANCER**

(71) Applicant: Fundación Centro Nacional de Investigaciones Oncológicas, 28029 Madrid (ES)
(72) Inventor: Heeschen, Christopher, 28029 Madrid (DE); Hermann, Patrick C., 28029 Madrid (DE); Lonardo, Enza, 28029 Madrid (IT)
(74) Representative: Pons Ariño, Angel

(57) **Abstract**

The present invention relates to a triple combination comprising a nodal/activin pathway inhibitor, a hedgehog pathway inhibitor and a chemotherapeutic agent and its use as a medicament for the treatment of cancer.

## Description

### FIELD OF THE INVENTION

The present invention relates to a triple combination comprising a nodal/activin pathway inhibitor, a hedgehog pathway inhibitor and a chemotherapeutic agent and its use as a medicament for the treatment of epithelial cancers.

### BACKROUND OF THE INVENTION

Epithelial cancers are among the most frequent causes of death. Especially pancreatic carcinomas are characterized by early metastatic spread and a pronounced resistance to chemotherapy and radiation. Despite extensive research activities in the field of tumour biology, there has hardly been any substantial progress within the past decades regarding therapeutic success. The introduction of the chemotherapeutic agent gemcitabine improved clinical response by reducing pain and loss of weight. As the median 5-year survival rate (1-4 %) and the median survival time are very low (5 months), the prognosis of patients with pancreatic cancer has remained poor.

Within the last years, it has been shown that stem cells play a decisive role in the development and progression of cancer, and that distinct populations of cells with stem cell properties may be essential for the development and perpetuation of various human cancers, including pancreatic cancer, colon cancer, lung cancer, breast cancer and brain tumour. According to the current consensus definition, a tumour cell that has the ability to self-renew, is exclusively tumorigenic, and is capable of producing the heterogeneous lineages of cancer cells that comprise the tumour fulfils the criteria of a cancer stem cell (CSC). Therefore, it is of the utmost importance to discover new treatment modalities leading to the elimination of CSC in order to eventually develop protocols for more successful treatment of cancer, especially of pancreatic cancer.

The treatment with the anti-metabolite gemcitabine, the first-line chemotherapeutic agent for the treatment of pancreatic cancer, has no detectable effect on CD133+ CSC. Cell cycle analyses even show that CSC immediately enter a state of cell divisions and therefore start the repopulation of the tumour immediately after the withdrawal of standard therapy. Thus, CD133+ tumour cells are highly enriched for the tumourigenic CSC fraction following gemcitabine therapy. Although *in vivo* therapy with gemcitabine resulted in local tumour growth in an orthotopic mouse model of xenotransplanted human pancreatic cancer, a significant enrichment of CD133+ cells in the residual tumour tissue could be demonstrated. Consequently, the withdrawal of gemcitabine will soon result in relapse, in most cases with an even more aggressive phenotype (Hermann P et al., 2007. Cell Stem Cell 1:313-323).

The accumulating evidence that standard therapy in many malignancies does not affect the suspected root of the disease, namely the CSC, emphasizes the urgent need for either developing targeted therapies against these cells or modifying current treatment modalities in order to be able to eliminate these cells. Considering that according to the CSC hypothesis only CSC are able to reproduce the heterogeneous lineages of cancer cells, attacking a tumour's CSC population seems to be the most promising approach for new developments. If a tumour is depleted of CSC, it loses its exclusive source for progression and metastasis, and should eventually degrade due to the limited life span of more differentiated tumour cells. As shown previously, a single CSC is able to reproduce an entire tumour (Zucchi, I., et al. 2007 PNAS 104: 10476 - 10481). Therefore, any CSC remaining after supposedly successful therapy would inevitably lead to tumour relapse. This undoubtedly shows that it is of utmost importance to develop novel therapies, which primarily target CSC and lead to their complete elimination.

Although pancreatic cancer has been the subject of increasing research efforts over the past decades, poor response to cytostatic and radiotherapy with subsequent dismal survival remains a hallmark of this devastating disease. Recent evidence suggests that pancreatic cancers harbour a distinct subpopulation of exclusively CSC. Most importantly, CSC have not only been identified as mediators of tumorigenicity but are also the source of resistance towards conventional chemotherapy and radiotherapy (Hermann P et al., 2007 Cell Stem Cell 1, 313-323). Therefore, as CSC are not only unaffected by current therapies but are also able to repopulate the disease and drive metastasis (Hermann P et al., 2008 Cell Cycle 7, 188-193), novel therapies capable of specifically targeting CSC, while leaving normal stem cells unaffected, are urgently needed.

Nodal and Activin are secreted proteins belonging to the TGF-β superfamily that were shown to be essential for human embryonic stem cell maintenance but their role in cancer is poorly understood. Hedgehog signalling is an essential pathway during embryonic pancreatic development, the deregulation of which has been implicated in several forms of cancer. Hedgehog signalling may also be an important mediator in human pancreatic carcinoma, as the inhibition of hedgehog signalling has been shown to induced apoptosis and block proliferation in a subset of the pancreatic cancer cell lines both *in vitro* and *in vivo* (Thayer SP et al., 2003 Nature, 425:851-6). The use of a combination of gemcitabine and a Hedgehog inhibitor in the treatment of pancreatic cancer has also been described by Olive et al. Science. 2009 12; 324 (5933): 1457-61 in the context of targeting the abundant stroma in pancreatic cancer. Combined therapies of a nucleoside analogue or antimetabolic agent like gemcitabine, and either a Nodal/Activin inhibitor or a Sonic Hedgehog (SHH) inhibitor and an mTOR-inhibitor, have been described in WO 2009112266. Also, the use of SB431542 for targeting the TGFβ pathway and erlotinib to enhance the efficacy of gemcitabine and cisplatin has been described by Sempere et al. Cancer Biol Ther. 2011, 12(3) 1-10.

### SUMMARY OF THE INVENTION

In a first aspect, the present invention refers to a pharmaceutical composition comprising a triple combination of: (a) a therapeutic effective amount of at least one nodal/activin pathway inhibitor, (b) a therapeutic effective amount of at least one hedgehog pathway inhibitor, and (c) a therapeutic effective amount of at least one chemotherapeutic agent or a pharmaceutically acceptable salt thereof.

In a second aspect, the present invention refers to a kit of parts comprising: (a) therapeutic effective amount of at least one nodal/activin pathway inhibitor, (b) a therapeutic effective amount of at least one hedgehog pathway inhibitor, and (c) a therapeutic effective amount of at least one chemotherapeutic agent or a pharmaceutically acceptable salt thereof, wherein the components are provided in a form which is suitable for sequential, separate and/or simultaneous administration.

In a third aspect, the present invention refers to the use of the pharmaceutical composition of the first aspect of the invention or the kit of parts of the second aspect of the invention, for use as a medicament, preferably for the treatment of epithelial cancer.

### BRIEF DESCRIPTION OF THE DRAWINGS

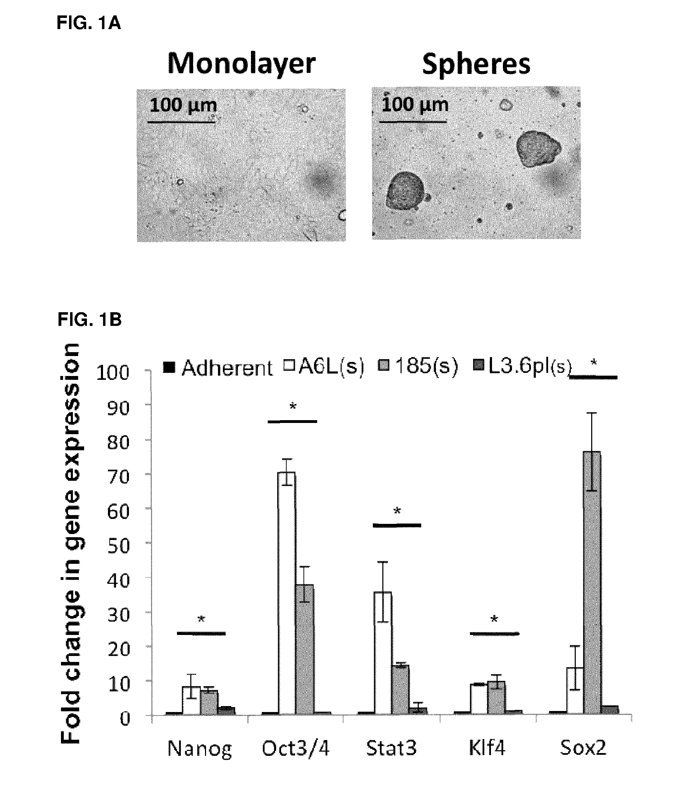

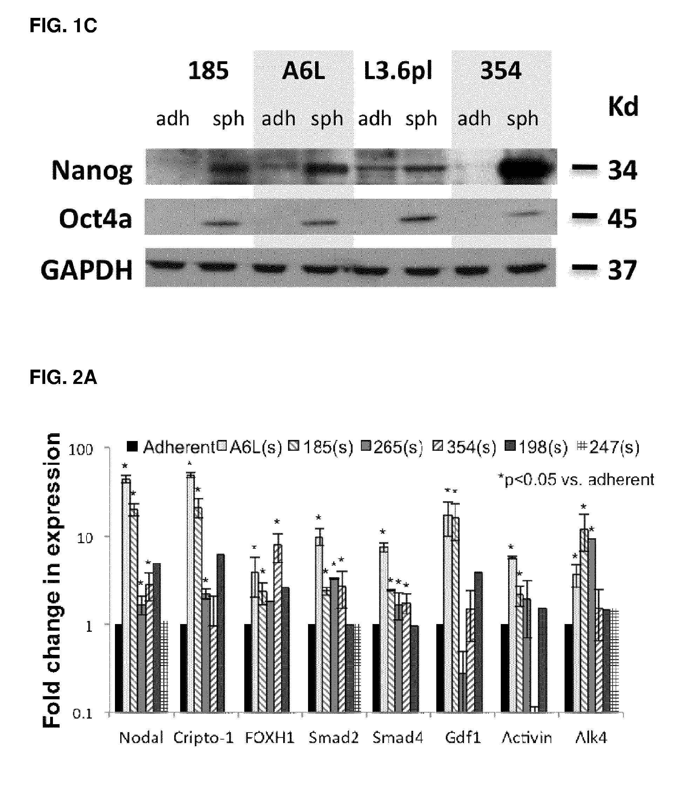

Figure 1. Validation of the cancer stem cell *in vitro* system. Sphere-derived pancreatic CSC express pluripotency markers. (A) Morphology of pancreatic cancer cells derived from xenografts and fresh human tissue grown as monolayers or spheres (enriching for cancer stem cells). (B) QPCR analysis of stemness genes in adherent cells versus spheres. Data are normalized to GAPDH expression and are presented as fold change in gene expression relative to control (C=adherent cells). Data are mean ± SD, n≥3. * indicates p<0.05 vs. control. (C) Western blot analysis for Nanog and Oct3/4a in spheres compared to adherent cells. Parallel GAPDH immunoblotting and signal quantification was performed by densitometry. ADU: arbitrary densitometric units.

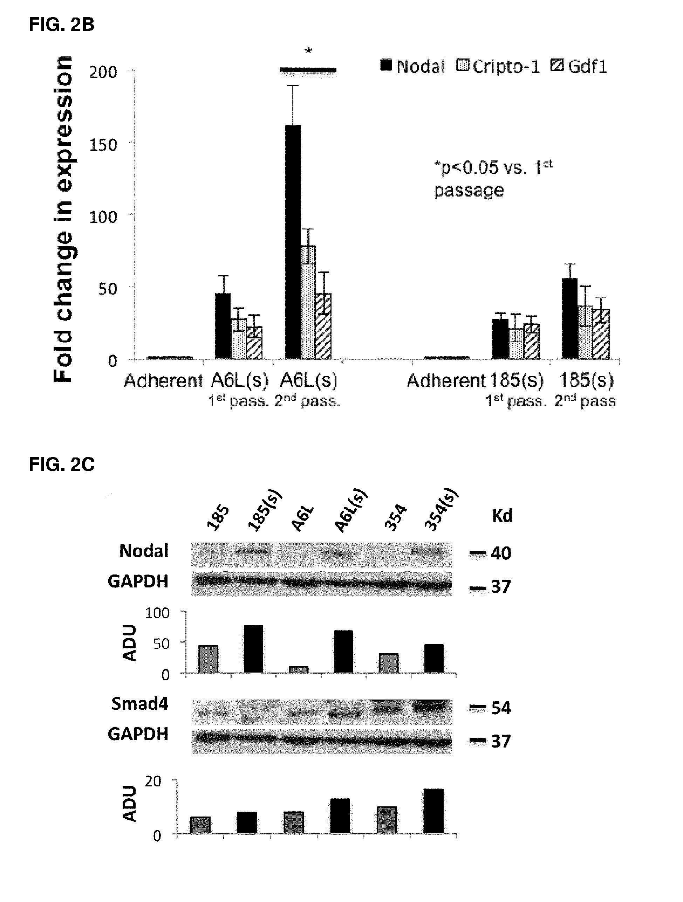

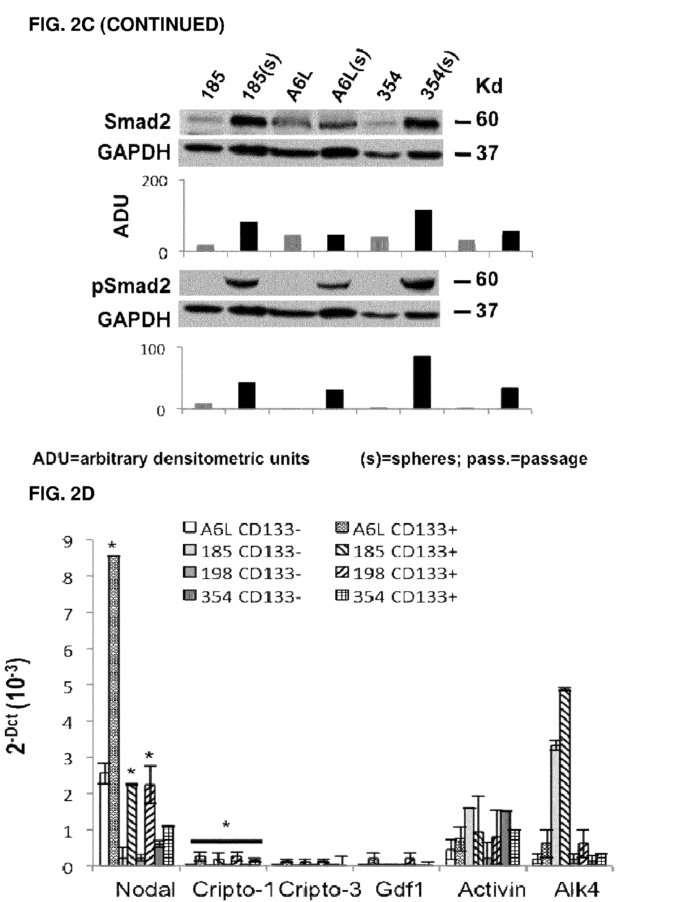

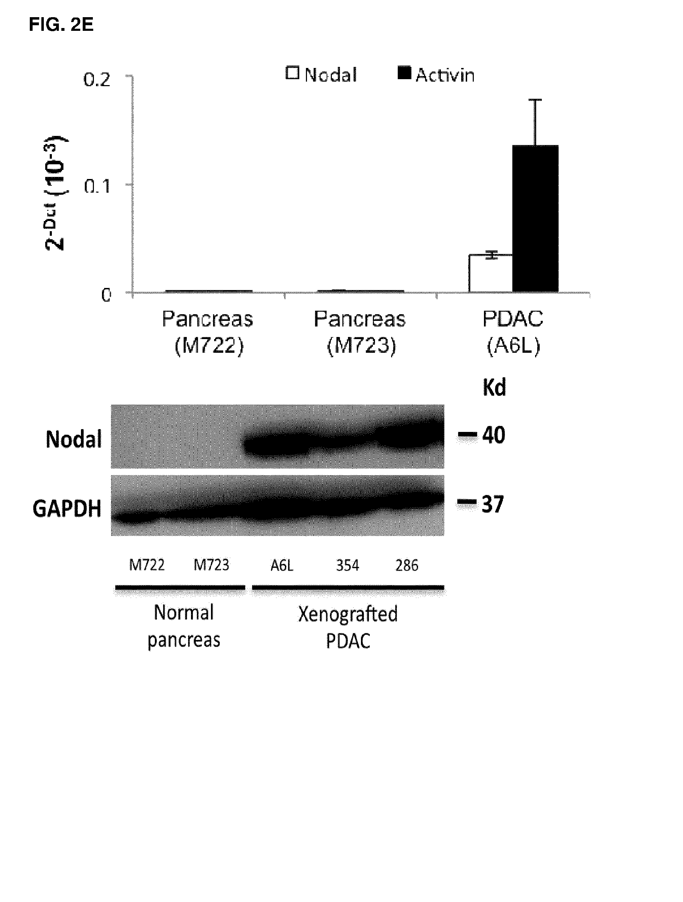

Figure 2. Components of the Nodal signalling pathway are specifically overexpressed in pancreatic CSC. QPCR analysis of Nodal signalling-associated genes in adherent cells versus spheres in first (A) and second passages (B). Data are normalized to GAPDH expression and are presented as fold change in gene expression relative to the control (C=adherent cells). Data are mean ± SD, n≥3. * indicates p<0.05 vs. control. (C) Western blot analysis for Nodal, Smad4, Smad2 and pSmad2 proteins in adherent cells versus spheres. Parallel GAPDH immunoblotting was performed and signal quantification was performed by densitometry. ADU are arbitrary densitometric units. (D) QPCR analysis for Nodal signalling-associated genes in CD133+ cells versus CD133- cells. Data are normalized to GAPDH and presented as mean ± SD, n≥3. * indicates p<0.001. (E) Lack of expression of Nodal in normal pancreas (M722 and M723) as opposed to pancreatic cancers (PDAC) as demonstrated by QPCR (upper panel) and western blot (lower panel).

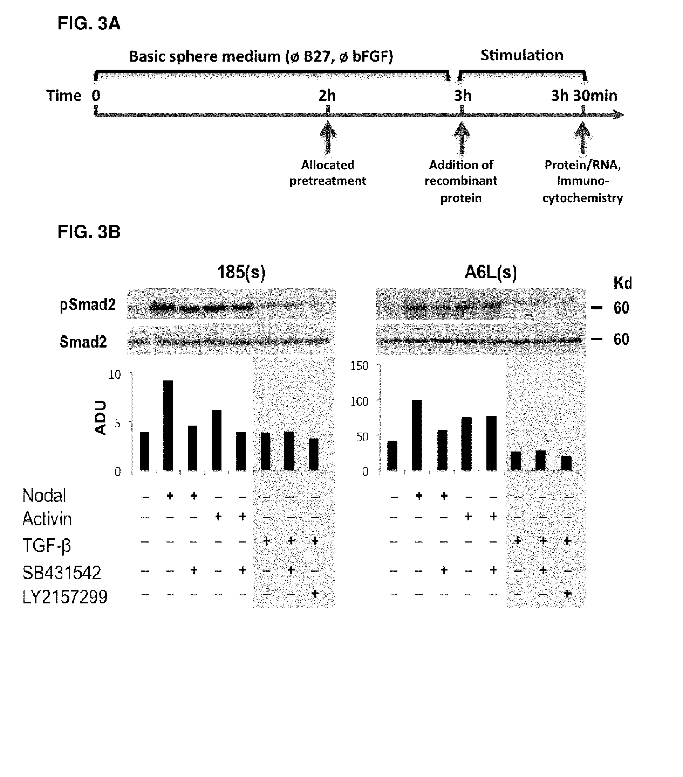

Figure 3. Nodal signalling is functionally active in pancreatic CSC. (A) Schematic illustration of Smad2 phosphorylation assay: cells were starved in basic medium for 3h and stimulated or unstimulated for another 30 min with rNodal or rActivin alone or in combination with SB431542. After the stimulation molecular and histological analyses were performed. (B) Western blotting for pSmad2 and Smad2 after treatment with rNodal (300 ng/ml), rActivin (100 ng/ml), rTGF-β (10 ng/ml), ALK4/5/7 inhibitor SB431542, or ALK5 inhibitor LY2157299 (20 µM) alone or in the indicated combinations. ADU are arbitrary densitometric units.

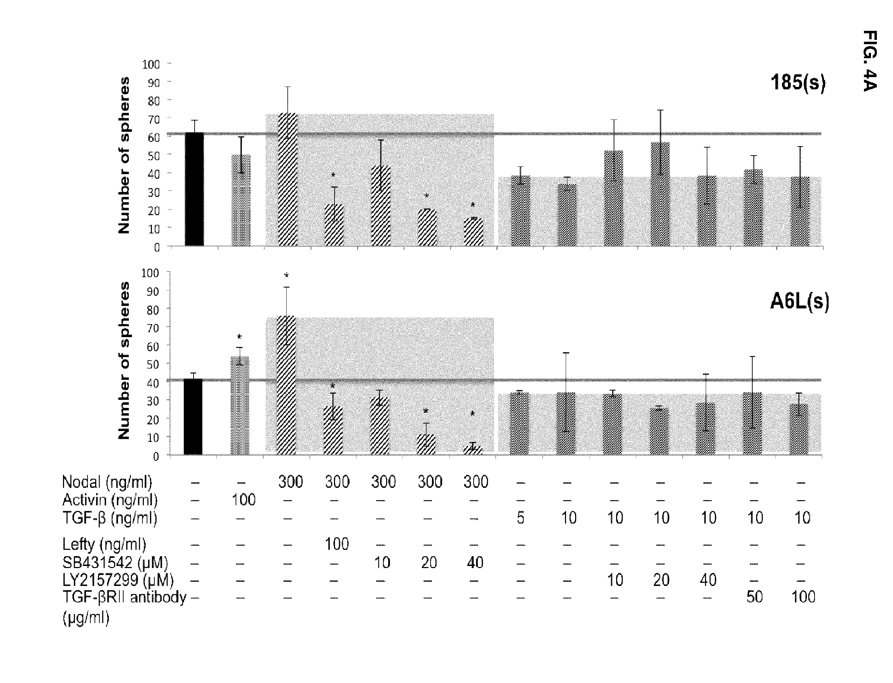

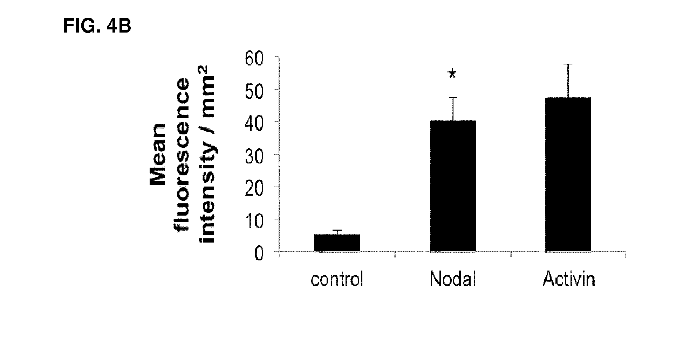

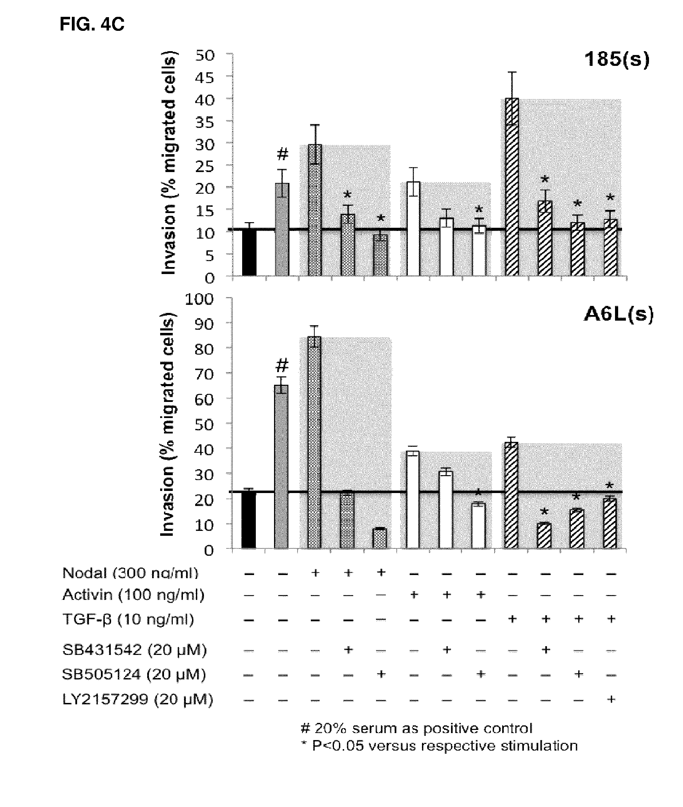

Figure 4. Nodal enhances proliferation and invasion of cancer stem cells. (A) Sphere formation assay for A6L and 185 cells after treatment with rNodal, rActivin, rTGFβ, rLefty, rFollistatin, SB431542 (Alk4/7 inhibitor), LY2157299 (Alk5 inhibitor) and TGFβRII neutralizing antibody. (B) Detection of Nanog positive cells by means of a RFP-tagged reporter construct for Nanog. Cells were treated with vehicle control, rNodal or rActivin. * indicates p< 0.05 (C) Cell invasion assay for 185 and A6L. Percentage of cell numbers in the lower chamber/total numbers of applied cells are presented. Cells were treated with rNodal, rActivin, rTGFβ, SB431542 (Alk4/7 inhibitor), SB505124 (Alk4/5/7 inhibitor) and LY2157299 (Alk5 inhibitor). * indicates p<0.05 vs. control.

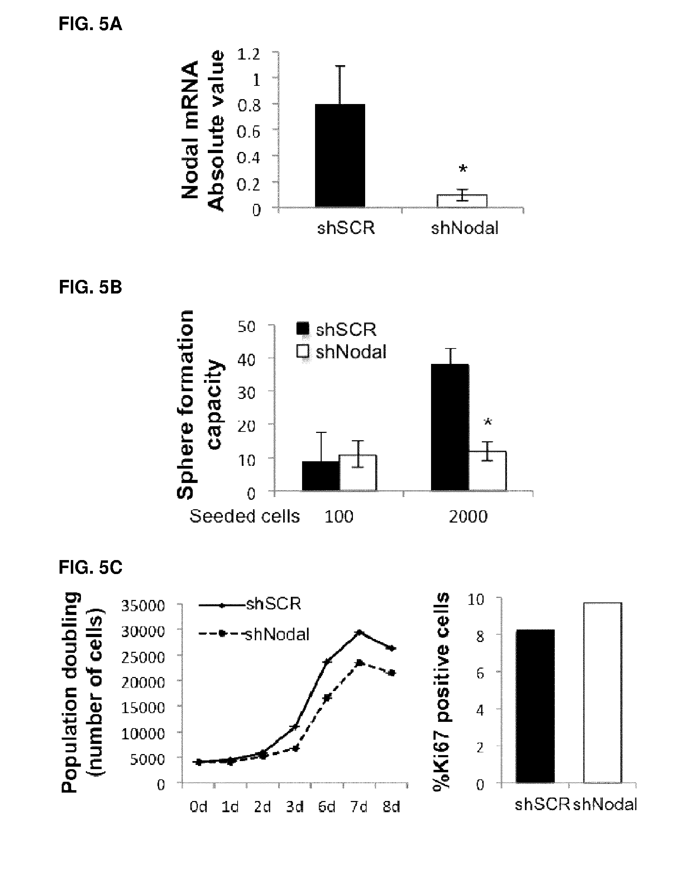

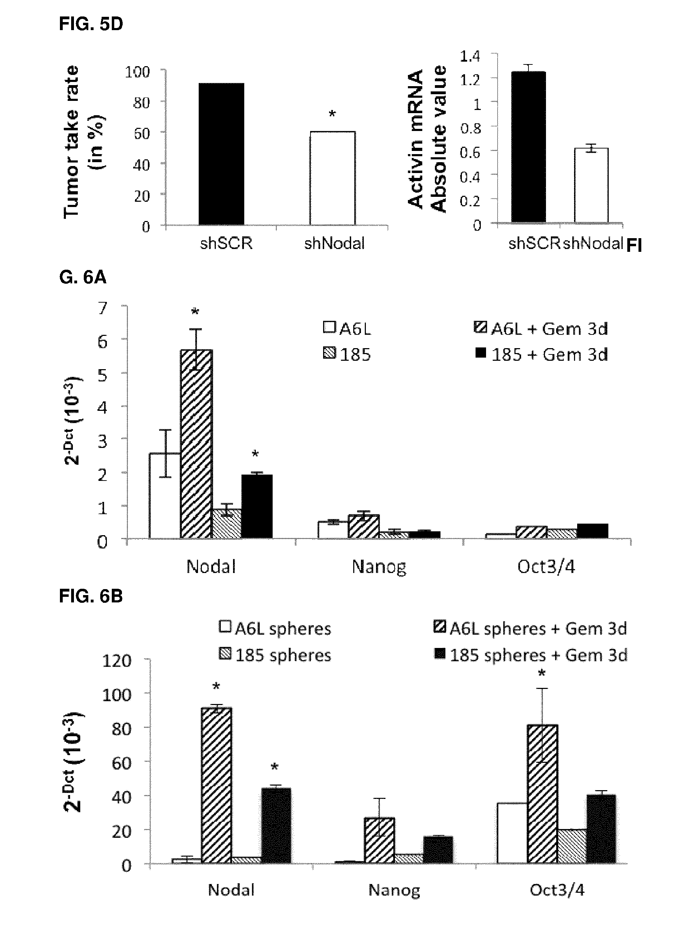

Figure 5. Knockdown of Nodal reduces self-renewal capacity of pancreatic CSC. (First row) (A) Knockdown of Nodal was demonstrated by qPCR (left) for ShNodal and ShScramble clones. GAPDH levels indicate equal sample loading. (B) Sphere formation capacity for ShScramble and ShNodal. (C) Proliferation of adherent (=differentiated) cancer cells was not affected as demonstrated by population doubling and Ki67 staining. (D) Tumour take rate was significantly reduced and accompanied by reduced Activin mRNA levels for the shNodal group.

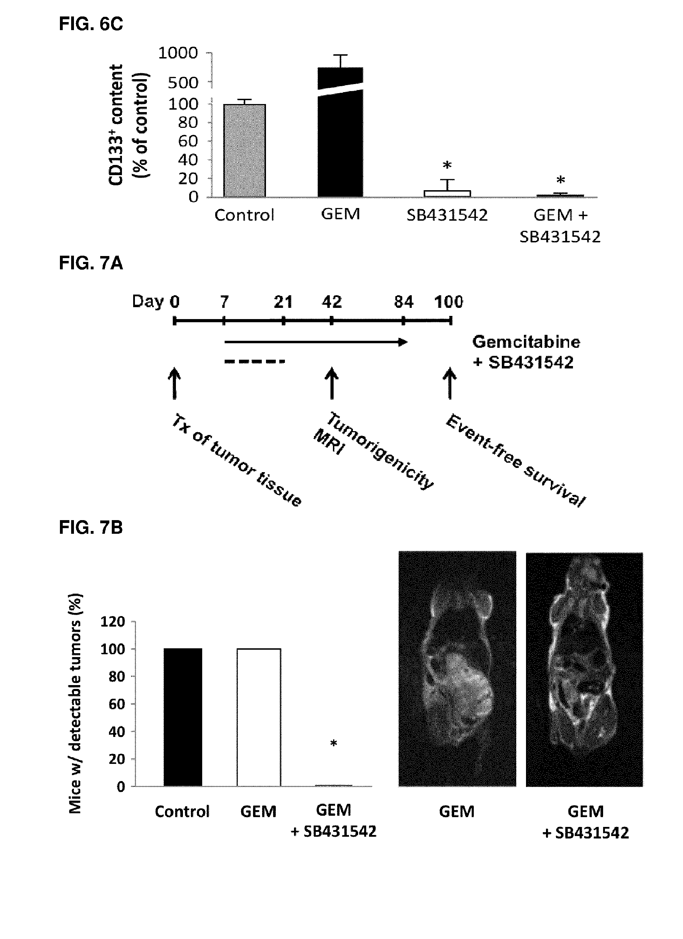

Figure 6. Nodal inhibition targets CD133 positive CSC. (A) QPCR analysis for Nodal, Nanog, and Oct3/4 genes in A6L and 185 adherent cells untreated or treated with gemcitabine (Gem). Data are normalized for GAPDH expression and are mean ± SD, n≥3. (B) QPCR analysis of Nodal, Nanog, and Oct3/4 genes in A6L and 185 spheres untreated or treated with Gem. Data were normalized for GAPDH expression and are mean ± SD, n≥3. (C) Quantification of CD133 expression in respective groups, n=3.

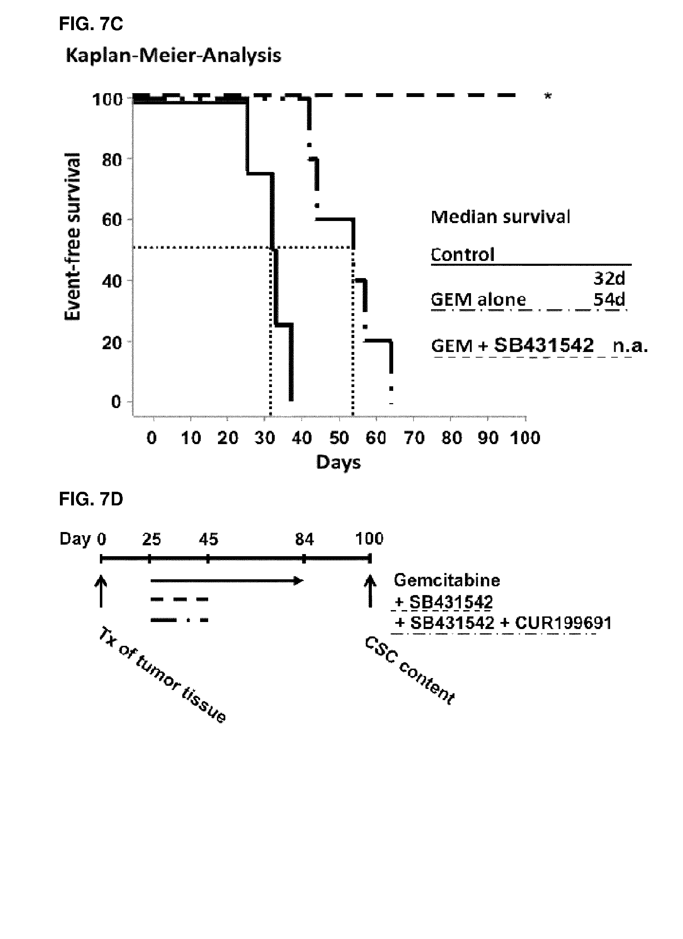

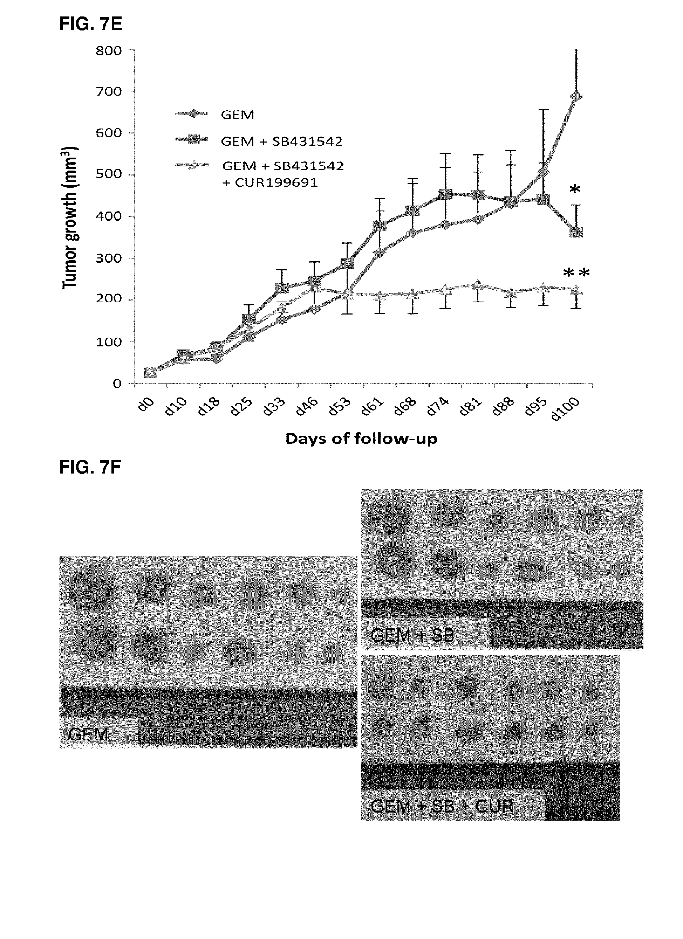

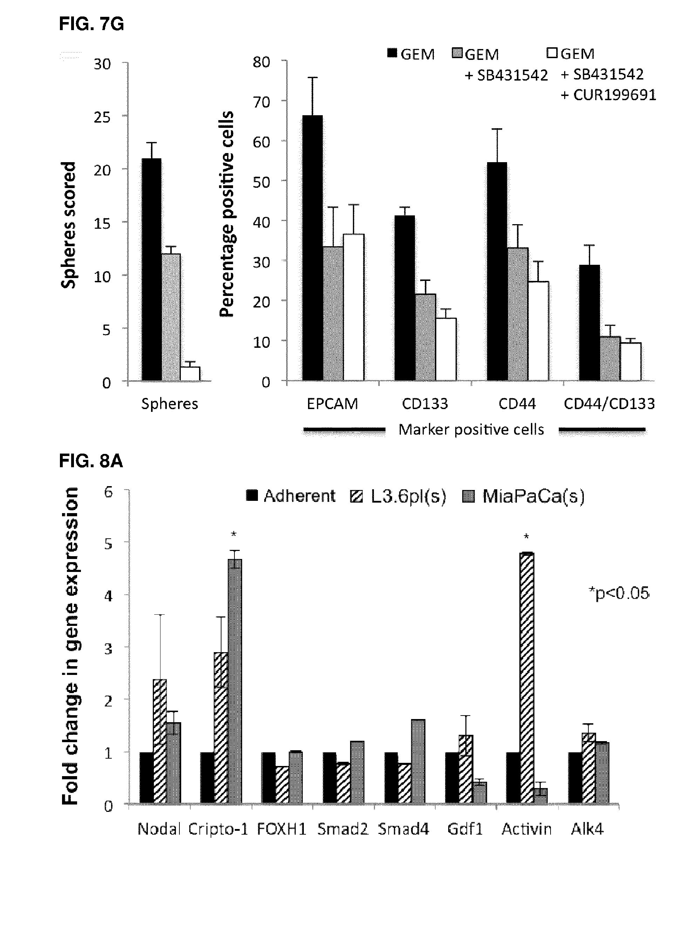

Figure 7. *In vivo* effects of Nodal/Activin inhibition on established pancreatic cancers using a pancreatic cancer cell line. (A) Experimental setup for *in vivo* experiments using L3.6pl cells. (B) Tumour take-rate (left) and representative MRI pictures (right) of treated pancreatic cancers. * indicates p<0.05 vs. control. (C) Kaplan-Meier-Analysis depicting cumulative survival of respective treatment groups. * indicates p<0.05 vs. GEM alone. (D) Experimental setup for *in vivo* experiments using primary human pancreatic cancer tissue. (E) Tumour growth is depicted for the respective treatment groups. (F) Explanted tumours on day 100 are shown. (G) On day 100, tumours from the different groups were digested and analysed for their respective sphere formation capacity (left panel) and cell surface marker expression (right panel).

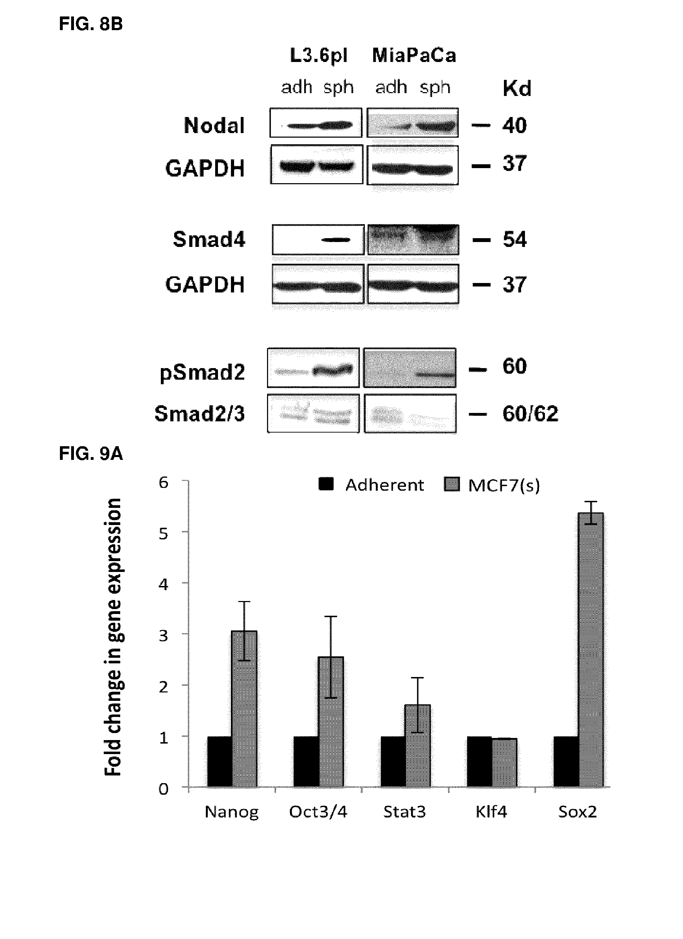

Figure 8. Components of the Nodal signalling pathway are also overexpressed in pancreatic CSC derived from established cell lines. (A) QPCR analysis of Nodal signalling-associated genes in adherent cells versus spheres in established pancreatic cancer cell lines as indicated. Data are normalized to GAPDH expression and are presented as fold change in gene expression relative to the control (C=adherent cells). Data are mean±SD, n≥3. (B) Western blot analysis for Nodal, Smad4, pSmad2 and Smad2/3 proteins in adherent cells versus spheres. Parallel GAPDH-immunoblotting was performed and signal quantification was performed by densitometry.

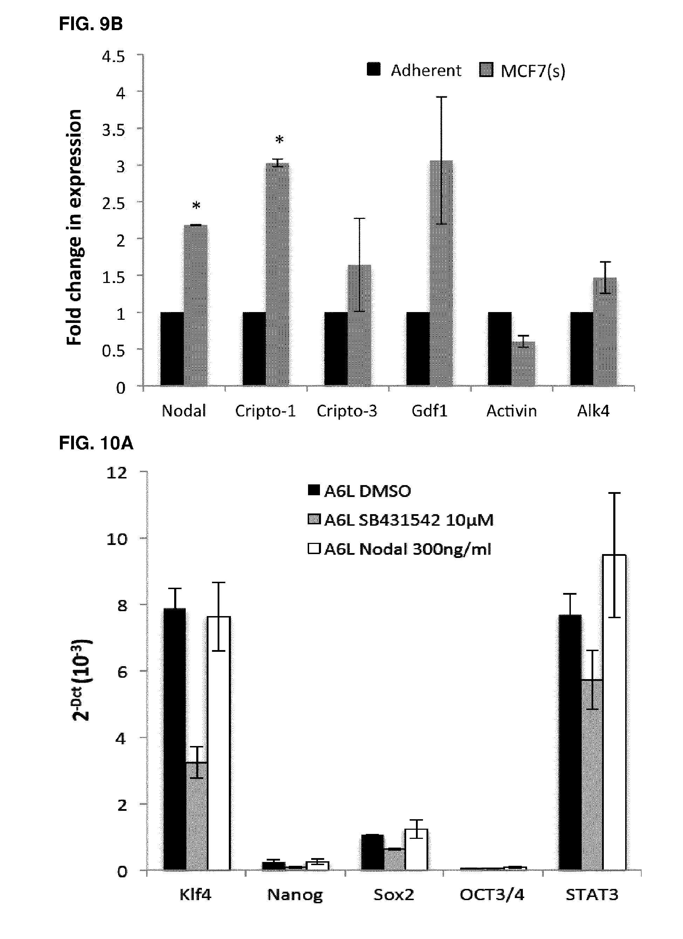

Figure 9. Expression pattern for pluripotency genes and Nodal pathway-associated genes in MCF7 breast cancer cells. (A) QPCR analysis of stemness genes in adherent cells versus spheres. Data are normalized to GAPDH expression and are presented as fold change in gene expression relative to control (C=adherent cells). Data are mean±SD, n≥3. (B) QPCR analysis of Nodal signalling-associated genes in adherent cells versus spheres. Data are normalized to GAPDH expression and are presented as fold change in gene expression relative to the control (C=adherent cells). Data are mean±SD, n≥3.

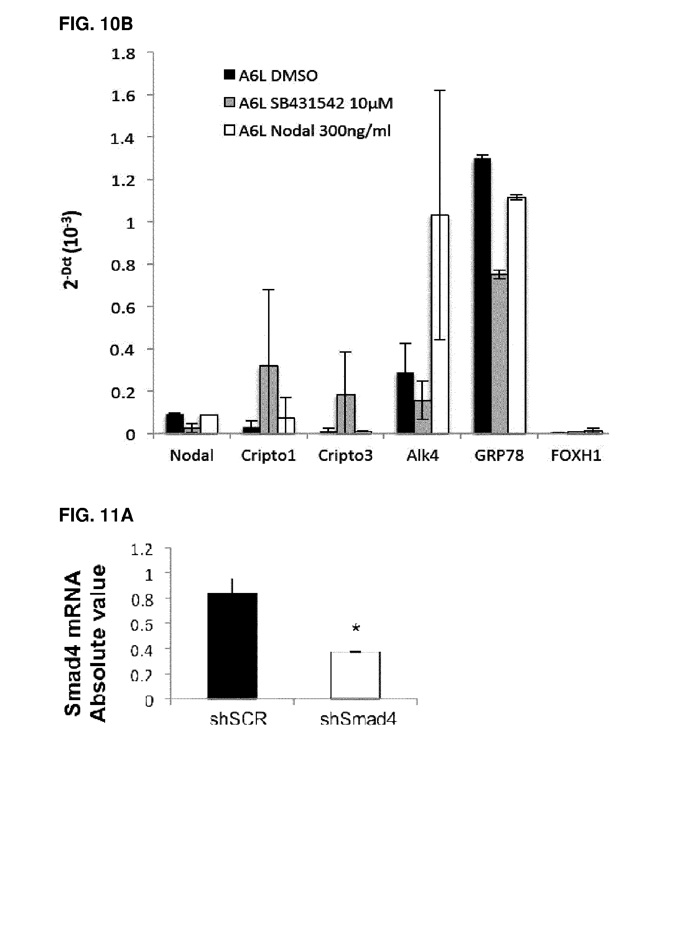

Figure 10. Modulation of the expression pattern of pluripotency genes and Nodal pathway-associated genes. (A) QPCR analysis of stemness genes in adherent cells treated as indicated. Data are presented as absolute gene expression normalized to GAPDH expression. Data are mean±SD, n≥3. (B) QPCR analysis of Nodal signalling-associated genes in adherent cells treated as indicated. Data are presented as absolute gene expression normalized to GAPDH expression. Data are mean±SD, n≥3.

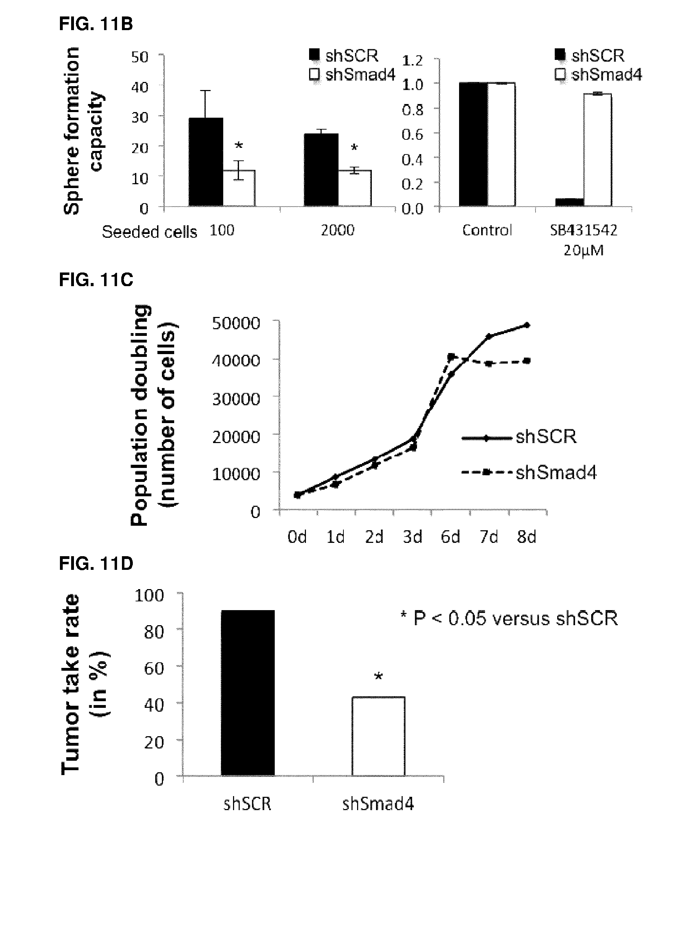

Figure 11. Sphere formation capacity depends on Smad4 signalling. (A) Knockdown of Smad4 was demonstrated by QPCR (left) for ShSmad4 and ShScramble clones (right). GAPDH levels indicate equal sample loading. (B) Sphere formation capacity for ShScramble and ShSmad4 (left). Response to treatment with the Alk4/7 inhibitor SB431542 (right). * indicates p<0.05. (C) Proliferation of adherent (=differentiated) cancer cells was not affected as demonstrated by population doubling. (D) Tumour take rate was significantly reduced for the shSmad4 group.

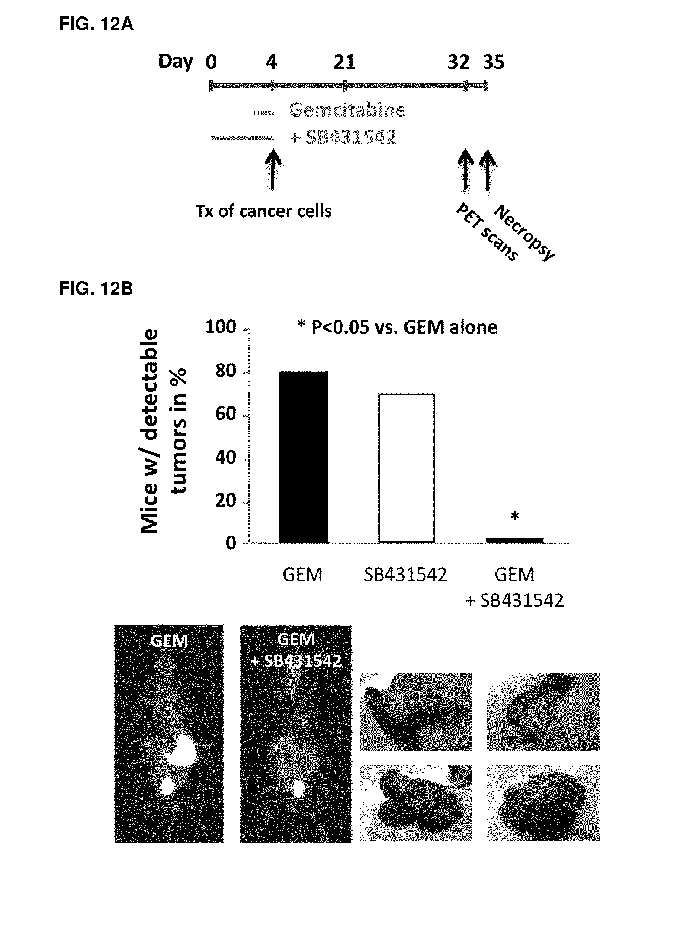

Figure 12. In vivo tumorigenicity of pretreated cells. (A) Experimental setup for in vivo validation of tumourigenicity of pretreated L3.6pl cells. (B) Tumour take-rate (upper panel) representative PET imaging (lower left panel), representative macroscopic pictures of the pancreata and liver (lower right panel, arrows indicate metastases) of treated pancreatic cancers.

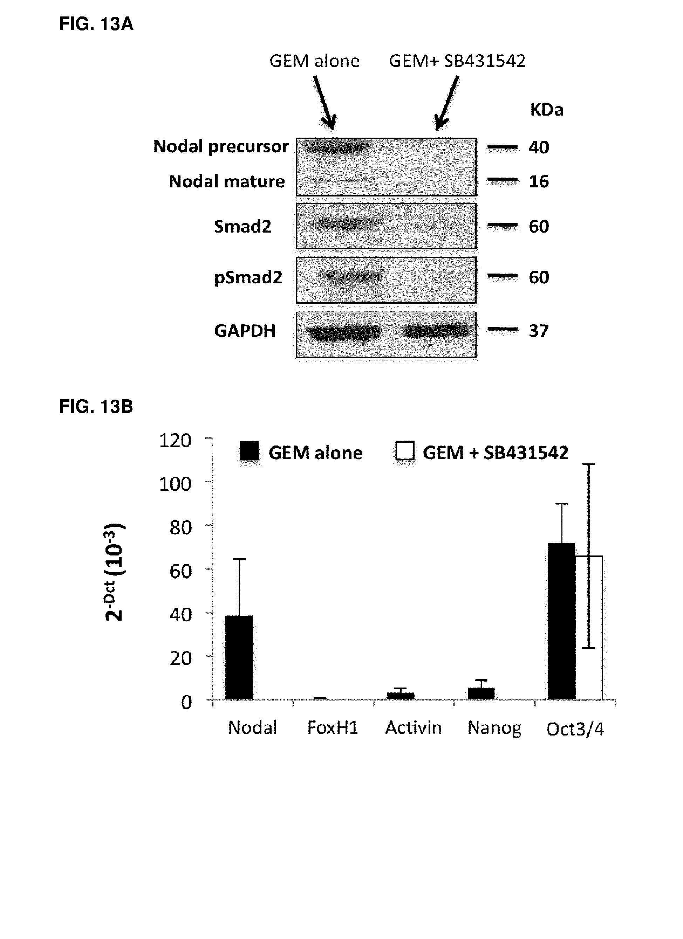

Figure 13. Validation of *in vivo* targeting of Nodal/activin inhibition using established primary pancreatic cancer xenografts. (A) For target validation, tumours were explanted and expression of key pathway members was assessed by Western blotting and (B) QPCR.

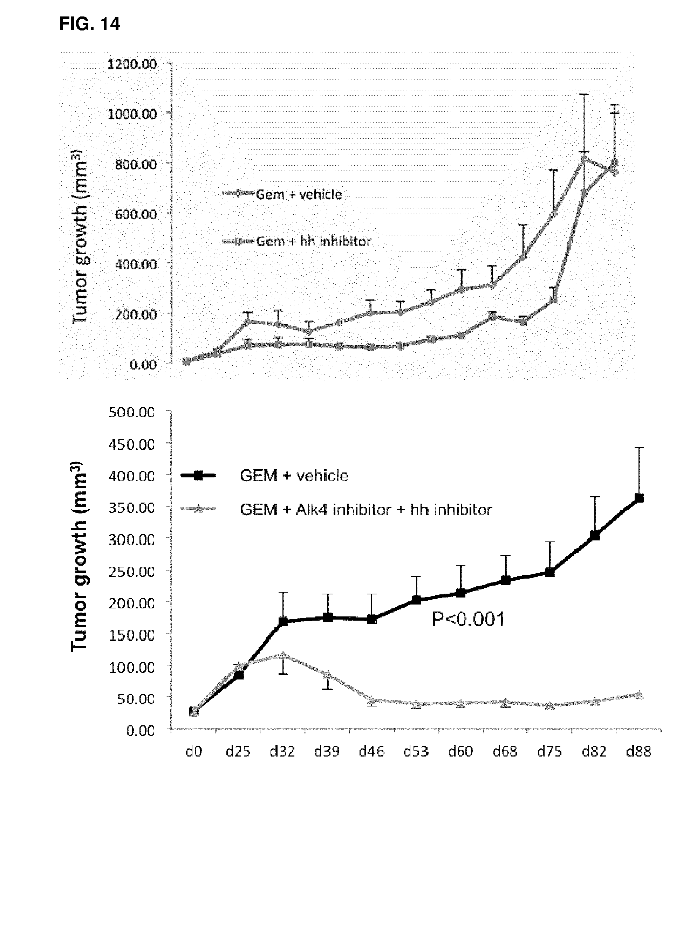

Figure 14. Inhibition of the Hedgehog pathway alone does not affect tumour progression (upper panel), while in combination with the Alk4/7 inhibitor SB431542 long-time survival can be achieved (lower panel). Tumours treated with gemcitabine alone or in combination with the hedgehog (hh) pathway inhibitor CUR199691 showed similar growth patterns (upper panel). Addition of the Alk4/7 inhibitor SB431542 resulted in tumour regression and long-term survival of the mice (lower panel; n=8-1 0 per group).

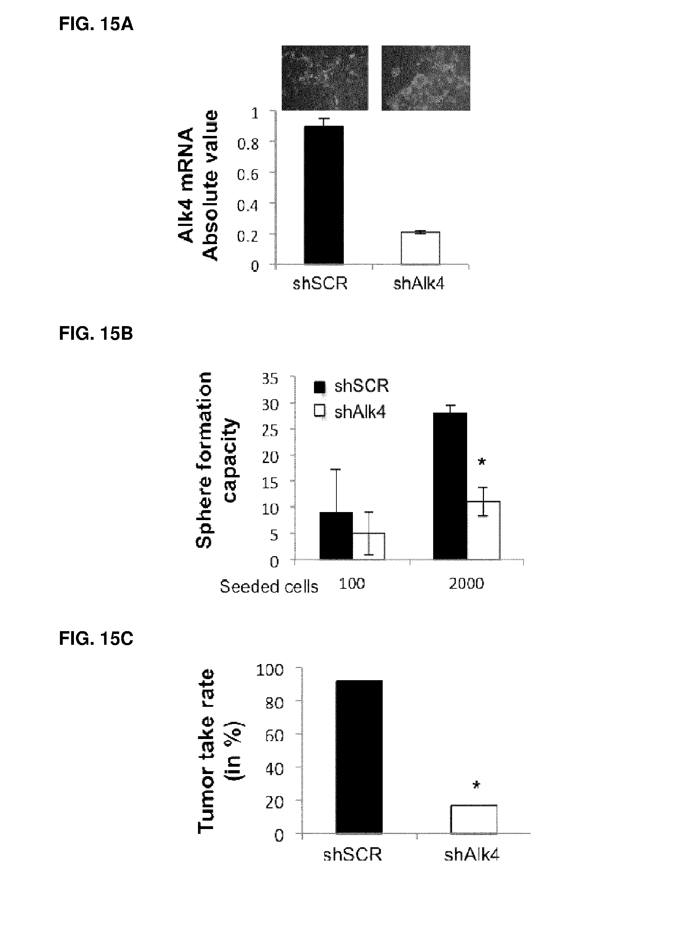

Figure 15. Genetic targeting of the Nodal/Activin signalling pathway abrogates cancer stem cells. (A) Lentivirally transduced cell cultures for scrambled shRNA (shSCR) or Alk4 shRNA (shAlk4) were FACSorted to highest purity based on GFP expression (representative images given in upper panel). Comparison of mRNA expression levels showed efficient knockdown of Alk4. (B) Sphere formation capacity was significantly reduced in Alk4 knockdown cells. (C) *In vivo* tumorigenicity was significantly reduced for Alk4 knockdown cells. Those small tumours that actually formed were virtually depleted for CD133+ cells in Alk4 knockdown tumours versus control.

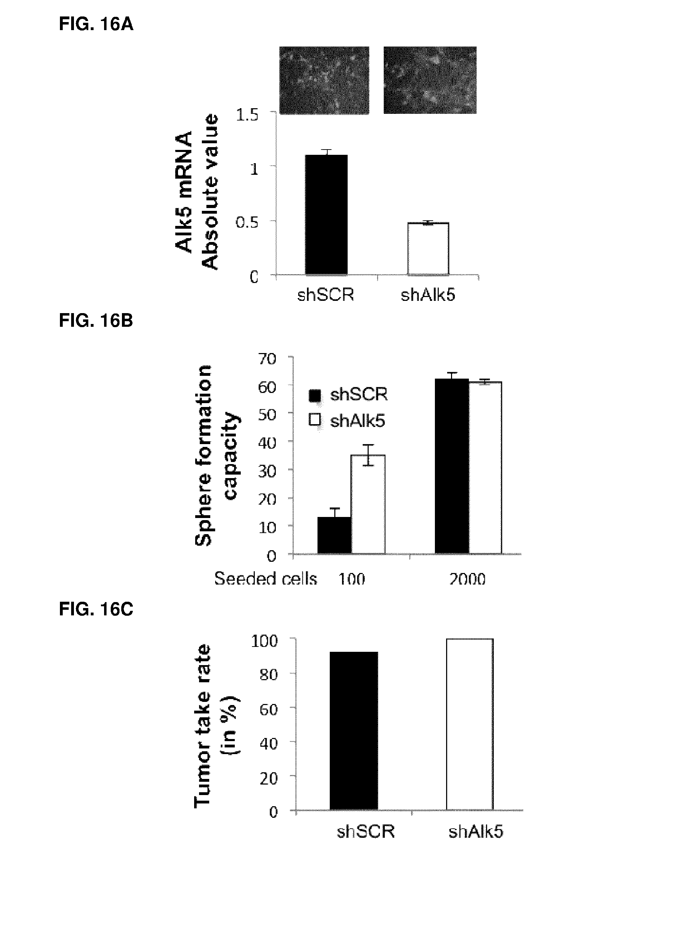

Figure 16. Genetic targeting of the TGFβ signalling pathway did not affect cancer stem cells. (A) Lentivirally transduced cell cultures for scrambled shRNA (shSCR) or Alk5 shRNA (shAlk5) were FACSorted to highest purity based on GFP expression (representative images given in upper panel). Comparison of mRNA expression levels showed efficient knockdown of Alk5. (B) Sphere formation capacity was not altered in Alk5 knockdown cells. (C) *In vivo* tumorigenicity was not reduced for Alk5 knockdown cells. The number of CD133+ cells in Alk5 knockdown tumours were not reduced as compared to controls.

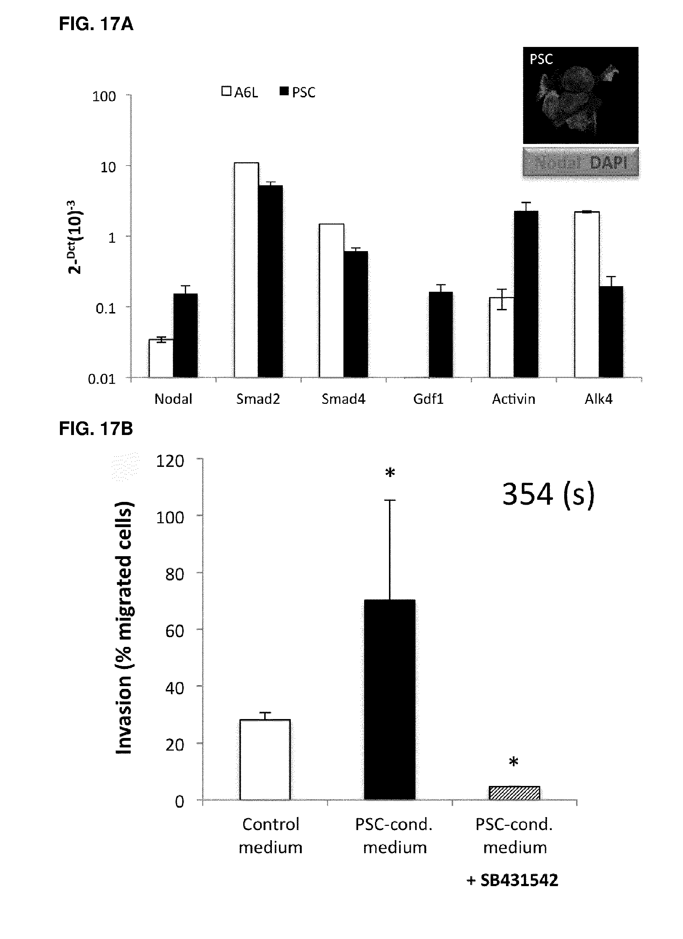

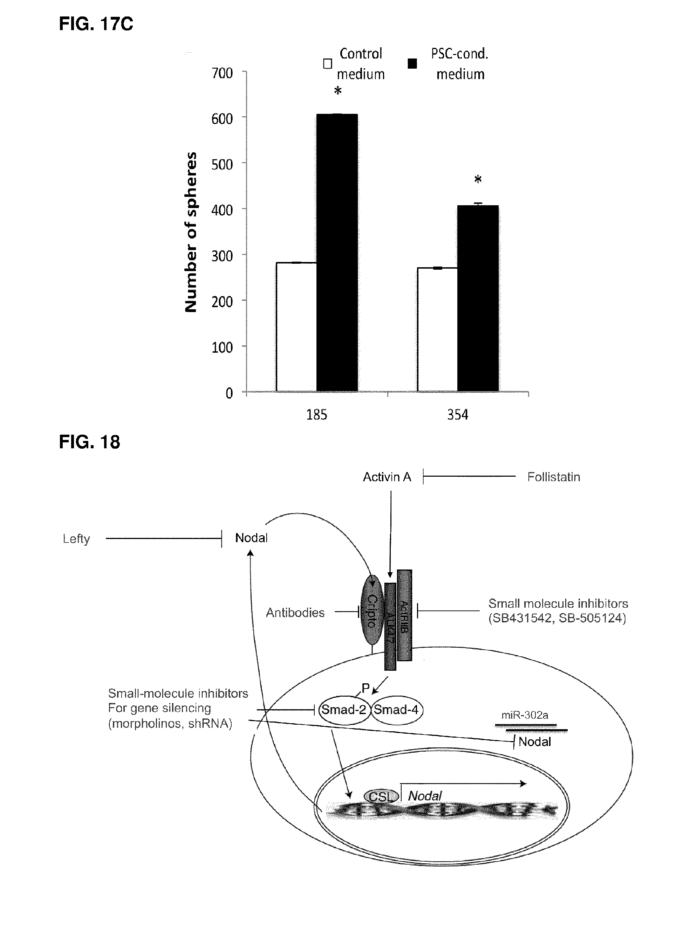

Figure 17. (A) QPCR analysis of Nodal/Activin signalling-associated genes in A6L cancer cells versus pancreatic stellate cells (PSC) illustrating strong expression of Nodal/Activin signalling-associated genes in PSC. Data are normalized for GAPDH expression and are mean ±SD, n≥3. (B) Invasion of sphere-derived cells in presence or absence of conditioned medium from PSC cells in the lower compartment of Matrige™-coated Boyden chambers. For preparation of PSC-conditioned medium, PSC were grown to 80% confluency in full medium (RPMI containing 10% FBS and antibiotics), which was then replaced by RPMI medium containing only 1% FBS and cultured for another 72 hours. Cell invasion was abrogated by the Nodal/Activin signalling pathway inhibitor SB431542. (C) Sphere formation capacity of 185 and A6L cells in the presence of PSC-conditioned DME/F12 medium or control medium in ultralow adhesion 24-well plates showed enhanced sphere formation for PSC-conditioned medium as an indication of paracrine activation of CSC by Nodal/Activin secreted from PSC.
Figure 18. Scheme of the Nodal/Activin pathway and exemplary ways to inhibit its signalling. The Nodal/Activin pathway can be targeted at different levels. First, the two ligands Nodal and Activin can be inhibited by their endogenous inhibitors Lefty (against Nodal) and Follistatin (against Activin). Secondly, small molecule inhibitors such as the Alk4/7 inhibitor SB431542, which was used in the present study, could be used to target their common receptor Alk4/7. Thirdly, as Cripto is an essential co-factor for Nodal, the inhibition of Cripto by specific antibodies represents an interesting option. Fourthly, targeting the downstream signalling molecules Smad2/3 or Smad4 is an efficient method to shut down this pathway. Finally, Nodal expression can be directly targeted by the use of microRNA302, which interferes with the ribosomal translation of Nodal mRNA.

### DEFINITIONS

As used herein, the term "therapeutic effective amount" refers to the quantity of a component, which is sufficient to yield a desired therapeutic response without undue adverse side effects (such as toxicity, irritation, or allergic response) commensurate with a reasonable benefit/risk ratio when used in the manner of this disclosure. By "therapeutically effective amount" is meant an amount of a compound of the present disclosure effective to yield the desired therapeutic response, for example, a reduction in tumour growth or in tumour size. The specific therapeutic effective amount will vary with such factors as the particular condition being treated, the physical condition of the patient, the type of mammal or animal being treated, the duration of the treatment, the nature of concurrent therapy (if any), and the specific formulations employed and the structure of the compounds or its derivatives.

As used herein, the term "consisting essentially of" means that the composition may comprises further features, but those features do not add materially any surprising technical effect.

As used herein, the term "nodal/activin pathway inhibitor" refers to a compound that inhibits Nodal and/or Activin and/or other ligands signalling through the same signalling cascade (Fig. 18). Nodal as well as Activin are both members of the Transforming Growth Factor TGFβ superfamily and play an essential role in embryonic development, particularly for maintaining the pluripotency of embryonic stem cells. Nodal and Activin signalling is both mediated through the receptors Activin receptor Like Kinase (ALK) 4 and 7 resulting in activation of Smad 2/3 by phosphorylation

Accordingly, "a nodal/activin pathway inhibitor" is defined as a compound inhibiting preferably the Nodal pathway and/or preferably the Activin pathway, either by inhibiting the ALK receptors, especially ALK 4 or 7, or by being Nodal antagonists or Activin antagonists. Such "nodal/activin pathway inhibitor" includes both inhibitors of Nodal and/or inhibitors of Activin, as both inhibitors are effective in the inventive context. Examples of such "nodal/activin pathway inhibitor" include SB431542, SB-505124, lefty, follistatin, cerberus, cripto binding protein, coco-protein, nicalin protein and nomo protein.

SB431542 and SB-505124 are known inhibitors of Nodal and of Activin acting on the TGFβ family receptors ALK4, 5 and 7 and are available through commercial sources (Sigma).

Lefty proteins, particularly Lefty1, are extracellular antagonists of Nodal. Lefty proteins are involved in embryogenesis and left-right patterning, e.g. assigning differences between the left and right sides, including heart and lung positioning. They specifically regulate the degree of left-right asymmetry during vertebrate development by controlling the spatiotemporal influence of the Nodal protein. Mutations in these genes cause incorrect positioning of these organs (e.g., situs invertis). Known Lefty proteins include Lefty 1 and 2. Lefty1 in the ventral midline prevents the Cerebrus (paracrine factor or "Caronte") signal from passing to the right side of the embryo. The role of Lefty1 is to restrict the expression of Lefty2 and Nodal to the left side, and to prevent Lefty2 or Nodal to encode a signal for 'leftness'. Lefty2 serves as a feedback inhibitor to restrict the range of nodal signalling during establishment of the left-right axis. It is related to Accession No. NP_066277.1

Follistatin is a single chain autocrine glycoprotein found to be ubiquitous within the body of nearly all higher animals that is the product of a single gene. It was initially isolated from follicular fluid and was identified as a protein fraction that inhibited Follicle-stimulating hormone (FSH) secretion from the anterior pituitary, and so was known as FSH-suppressing protein (FSP). Since then its primary function has been determined to be the binding and bioneutralization agent of members of the TGFβ superfamily, with primary focus on Activin, which enhances secretion of FSH in the anterior pituitary. It is related to Accession No. NP_006341.1.

Cerberus is an extracellular protein that belongs to a subclass of the cysteine-knot superfamily. Cerberus-like proteins act as trifunctional growth-factor antagonists, which bind and block not only Nodal but also Wnt and BMP ligands. However, a truncated form of Cerberus (Cer-short) specifically blocks Nodal signals. Although Cerberus expression does not completely conform to Nodal signalling activity, it can be induced by Nodal signals in a subset of cells, thus potentially serving as a region-specific feedback inhibitor of Nodal signalling (Piccolo et al. (1999) Nature 397, 707±710). It is related to Accession No. NP_005445.1.

Cripto is a co-receptor of Nodal and has been described inter alia by Lonardo et al. 2010 Stem Cell 28:1326-1337. It is related to Accession No. NP_035692.2. Cripto binding protein is a protein that binds to cripto and blocks the signalling cascade (Fig. 18). A cripto binding protein can be, but is not limited to, a specific antibody with blocking capacity.

The Coco-Protein (also described as 51-B6) is described inter alia by Bell et al. (2003) Development 130, 1381-1389. It is related to Accession No. NP 001092196.

The Nicalin-Protein is described/mentioned inter alia by Haffner et al. (2004) EMBO 15; 3041-3050 and Hafner et al. (2007), J. Biol. Chem. 282(14); 10632-8. It is related to Accession No. NP 064555.

The Nomo-Protein/s (also known as pM5) is described/mentioned inter alia by Haffner et al. (2004) EMBO 15; 3041-3050 and Hafner et al. (2007), J. Biol. Chem. 282(14); 10632-8. It/they are related to Accession Nos. AAH65535, NP 001004067, N P 001004060, N P 775885.

A nodal/activin pathway inhibitor can be identified performing an assay to quantify Smad-2 phosphorylation in cells expressing Alk receptors only or Alk receptor and Cripto, after the addition of the potential inhibitor. These assays have been described in all detail in Inman GJ et al. (2002) Mol Pharmacol. 62(1); 65-74. SB-431542 is a potent and specific inhibitor of transforming growth factor-β superfamily type I activin receptor-like kinase (ALK) receptors ALK4, ALK5, and ALK7.

As used herein, the term "hedgehog pathway inhibitor" refers to a compound that inhibits Hedgehog signalling. Hedgehog is the extracellular component of the pathway and activates intracellular signals after binding to its specific receptor "Patched" (Ptch), a protein located on the cellular wall. After binding of Hedgehog to Patched a protein called "Smoothened" (SMO) becomes activated and thus induces transcription of target genes of the Hedgehog pathway. In the absence of Hedgehog the activity of Smoothened is suppressed by Patched and thus the target genes of the Hedgehog pathway are not expressed. The inhibition of the hedgehog pathway can be tested by studying the repression of the target gene Gli by qPCR, as described in Mueller TM et al. Gastroenterology 2009 137(3):1102-13.

Accordingly a "hedgehog pathway inhibitor" is defined as a compound targeting at least one component of the hedgehog signalling pathway, thus inhibiting its activity. Examples of hedgehog pathway inhibitors include CUR199691, CUR61414, cyclopamine, cyclopamine-KAAD, Jervine, Forskolin, SANT-1, Arsenic Trioxide (ATO), SIBI-C1, GDC-0449, IPI-926, Gant61, Gant58, Aktar01 1861, Aktar01 1332, Aktar01 1861 and Aktar30337.

CUR199691 and CUR61414 are small molecules inhibiting Smoothened and thereby blocking the hedgehog pathway and were developed by CURIS Inc., USA.

Cyclopamine (11-deoxojervine) is a natural occurring steroidal jerveratrum alkaloid influencing the balance between active and inactive SMO and is freely available through chemical suppliers.

Cyclopamine-KAAD (3-Keto-N-(aminoethyl-aminocaproyl-dihydrocinnamoyl) Cyclopamine) is a variant of Cyclopamine, also influencing the balance between active and inactive SMO and is freely available through chemical suppliers.

Jervine is another natural occurring steroidal alkaloid from veratrum against a SMO antagonist and is freely available through chemical suppliers.

Forskolin is a natural occurring labdane diterpene commonly used to raise the level of CAMP and is also freely available from chemical suppliers.

SANT-1 (N-[(3,5-dimethyl-1-phenyl-1H-pyrazol-4-yl)methylene]-4-(phenylmethyl)-1-piperazinamine) is a potent hedgehog pathway inhibitor as it antagonizes SMO activity, and is freely available through chemical suppliers.

Arsenic Trioxide (ATO) is a well-know derivative of arsenic available through medical suppliers.

SIBI-C1 is a small molecule inhibiting Smoothened and thereby blocking the hedgehog pathway and was developed by Siena Biotech, Italy.

GDC-0449 is a small molecule inhibiting Smoothened and thereby blocking the hedgehog pathway and was developed by Genentech, South San Francisco, California.

IPI-926 is a small molecule inhibiting Smoothened and thereby blocking the hedgehog pathway and was developed by Infinity, Massachusetts

Gant61 and Gant58 are pan-Gli (downstream target of the hedgehog pathway) inhibitors and commercially available from Sigma.

Actar0l 1861, Actar011332, Actar0l 1861 1 and Actar30337 are pan-Gli inhibitors developed by Actar AB, Sweden

As used herein, the term "chemotherapeutic agent" refers to cytotoxic antineoplastic agents, that is, chemical agents which preferentially kill neoplastic cells or disrupt the cell cycle of rapidly proliferating cells, used therapeutically to prevent or reduce the growth of neoplastic cells. Chemotherapeutic agents are also known as antineoplastic drugs or cytotoxic agents, and are well known in the art. As used herein, chemotherapy includes treatment with a single chemotherapeutic agent or with a combination of agents. In a subject in need of treatment, chemotherapy may be combined with surgical treatment or radiation therapy, or with other antineoplastic treatment modalities. "Chemotherapy" in the sense of this invention is defined as the use of a chemotherapeutic drug or active substance combination for the treatment of cancer or tumours or malign neoplasia respectively. Typically, the preferred chemotherapeutic agents are those which exhibit at least 50% of the activity of gemcitabine, more preferably at least 60% of the activity of gemcitabine, even more preferably 70%, 80% or 90% of the activity of gemcitabine, most preferably 95%, 96%, 97%, 98%, 99% or even 100% of the activity of gemcitabine. The activity of gemcitabine may be defined as its capability to inhibit or terminate DNA replication (or RNA synthesis) in normal human DNA replication. The preferred example of a chemotherapeutic agent for this invention is gemcitabine (IUPAC name: 4-amino-l-[3,3-difluoro-4-hydroxy-5-(hydroxymethyl) tetrahydrofuran-2-yl]- 1 H-pyrimidin- 2-one). Gemcitabine is a pyrimidine analog, marketed as Gemzar™, in which the hydrogens on the 2' carbons of deoxycytidine are replaced by fluorines.

Fluorouracil (5-FU or f5U) (IUPAC name: 5-fluoro-1H-pyrimidine-2,4-dione) is a pyrimidine analogue, which is used as a drug in the treatment of cancer. It principally acts as a thymidylate synthase inhibitor. Interrupting the action of this enzyme blocks synthesis of the pyrimidine thymidine, which is a nucleotide required for DNA replication. Thymidylate synthase methylates deoxyuridine monophoshate (dUMP) into thymidine monophosphate (dTMP).

Capecitabine (IUPAC name: pentyl[1-(3,4-dihydroxy-5-methyltetrahydrofuran-2-yl)-5-fluoro-2-oxo-1H-pyrimidin-4-yl]aminomethanoate) is a pyrimidine analogue, which acts as a prodrug, that is enzymatically converted to 5-fluorouracil in the tumour. There, it inhibits DNA synthesis and slows growth of tumour tissue. The activation of capecitabine follows a pathway with three enzymatic steps and two intermediary metabolites, 5'-deoxy-5-fluorocytidine (5'-DFCR) and 5'-deoxy-5-fluorouridine (5'-DFUR), to form 5-fluorouracil. Capecitabine is marketed under the trade name Xeloda.

Azathioprine (IUPAC name: 6-[(1-methyl-4-nitro-1H-imidazol-5-yl)sulfanyl]-7H-purine) is a purine analogue and a purine synthesis inhibitor, inhibiting the proliferation of cells.

Mercaptopurine (also called 6-Mercaptopurine, 6-MP or its brand name Purinethol) (IUPAC name: 3,7-dihydropurine-6-thione) is converted to the corresponding ribonucleotide. 6-MP ribonucleotide inhibits purine nucleotide synthesis and metabolism. This alters the synthesis and function of RNA and DNA. Mercaptopurine interferes with nucleotidee interconversion and glycoprotein synthesis.

As used herein, the term "pharmaceutically acceptable excipient" refers to a non-toxic, inert solid, semi-solid or liquid filler, diluent, encapsulating material or formulation auxiliary of any type. A pharmaceutically acceptable excipient is any excipient, which is relatively non-toxic and innocuous to a patient at concentrations consistent with effective activity of the active ingredient so that any side effects ascribable to the excipient do not vitiate the beneficial effects of the active ingredient. Pharmaceutically acceptable excipients are for example carriers, diluents, disintegrants, binders, lubricants, fillers, plasticizers, surfactants and wetting agents, film-forming agents and coating materials, and colouring agents, for example pigments.

As used herein, the expressions "concurrent administration", "simultaneous administration" or "administered simultaneously", mean that the compounds are administered at the same point in time or immediately following one another. In the latter case, the compounds are administered at times sufficiently close that the results observed are essentially indistinguishable from those achieved when the compounds are administered at the same point in time.

### DETAILED DESCRIPTION OF THE INVENTION

The present invention provides a triple combination for the treatment of cancer. The authors of the present invention have shown that the combination of a chemotherapeutic agent such as gemcitabine with a nodal/activin pathway inhibitor and a hedgehog pathway inhibitor reduces the tumorigenic capacity of cancer stem cells (CSC).

The authors of the present invention have proved that the treatment of *in vivo* tumours with the triple combination of the invention abolished the proliferative capacity of the CSC in the tumour. Therefore, the present invention provides a pharmaceutical composition with an enhanced chemotherapeutic effect of gemcitabine that overcomes the chemoresistance found in certain cancers, such as pancreatic cancer, particularly finding therapies that achieve a better *in vivo* result in reducing the number and size of the tumours.

Moreover, the pharmaceutical composition of the invention has a synergic effect on tumour growth, as it is clearly shown in figures 7E-F and 14. Both double combinations of gemcitabine with either a nodal/activin pathway inhibitor or a hedgehog pathway inhibitor showed poor results in the inhibition of tumour growth *in vivo*. Both double combinations, although reduced slightly the tumour size, showed patterns of tumour growth similar to that of gemcitabine alone in *in vivo* tumours. Unexpectedly, the triple combination of a chemotherapeutic agent such as gemcitabine with a nodal/activin pathway inhibitor and a hedgehog pathway inhibitor, caused an immediate inhibition of tumour progression and eventually translated into a long-term stable disease. A first aspect of the present invention is a pharmaceutical composition (hereafter referred as "composition of the invention") comprising a triple combination of:
a) a therapeutic effective amount of at least one nodal/activin pathway inhibitor,
b) a therapeutic effective amount of at least one hedgehog pathway inhibitor, and
c) a therapeutic effective amount of at least one chemotherapeutic agent or a pharmaceutically acceptable salt thereof.

A preferred embodiment of the first aspect of the present invention is a pharmaceutical composition consisting essentially of:
a) a therapeutic effective amount of at least one nodal/activin pathway inhibitor,
b) a therapeutic effective amount of at least one hedgehog pathway inhibitor, and
c) a therapeutic effective amount of at least one chemotherapeutic agent or a pharmaceutically acceptable salt thereof.

A preferred embodiment of the first aspect of the present invention is a pharmaceutical composition consisting of:
a) a therapeutic effective amount of at least one nodal/activin pathway inhibitor,
b) a therapeutic effective amount of at least one hedgehog pathway inhibitor, and
c) a therapeutic effective amount of at least one chemotherapeutic agent.

In a preferred embodiment of the first aspect of the invention, at least one nodal/activin pathway inhibitor is selected from the list comprising: SB431542, SB505124, lefty, follistatin, cerberus, cripto binding protein, coco-protein, nicalin protein and nomo protein. In a preferred embodiment of the first aspect of the invention, the nodal/activin pathway inhibitor is a ligand inhibitor such as follistatin or lefty. In a preferred embodiment of the first aspect of the invention, the nodal/activin pathway inhibitor is a receptor inhibitor, such as a blocking antibody against Cripto, SB431542 or SB505124. In a preferred embodiment of the first aspect of the invention, the nodal/activin pathway inhibitor is an ALK4/7 receptor inhibitor such as SB431542 or SB505124. In a preferred embodiment of the first aspect of the invention, the nodal/activin pathway inhibitor is a RNA interfering molecule such as morpholino, siRNA or shRNA, which inhibits Samd-2 or Smad-4. In a more preferred embodiment of the first aspect of the invention, the nodal/activin pathway inhibitor is SB431542.

In a preferred embodiment of the first aspect of the invention, at least one hedgehog pathway inhibitor is a smoothened inhibitor or a Gli inhibitor or a combination of both. In a preferred embodiment of the first aspect of the invention, at least one smoothened inhibitor is selected from the list comprising: CUR199691, CUR 61414, cyclopamine, cyclopamine-KAAD, Jervine, Forskolin, SANT-1, Arsenic Trioxide (ATO), SIBI-C1, GDC-0449 and IPI-926. In a preferred embodiment of the first aspect of the invention, at least one Gli inhibitor is selected from the list comprising: Gant61, Gant58, Aktar011861, Aktar01 1332, Aktar01 1861 and Aktar30337. In a more preferred embodiment of the first aspect of the invention, the hedgehog pathway inhibitor is CUR199691.

In a preferred embodiment of the first aspect of the invention, at least one chemotherapeutic agent is selected from the list comprising: gemcitabine, capecitabine, 5-FU, methotrexate, oxaliplatin, cisplatin, carboplatin, cyclophosphamide, doxorubicine, folic acid, retinoic acid, irinotecan, topotecan, leucovorin, Abraxane, erlotinib, paclitaxel, docetaxel, vincristine and its derivates, etoposide, teniposide, azathioprine, 6-mercaptopurine. In a more preferred embodiment of the first aspect of the invention, the chemotherapeutic agent is gemcitabine.

In the present invention, the administration of the composition of the invention can be complemented by anti-cancer radiotherapy. The anti-cancer radiotherapy may be carried out in any conventional manner as is known in the art, including external beam radiotherapy and implanted radioactive sources (such as temporarily implanted radioactive iodine sources to deliver high dose local therapy).

A preferred embodiment of the first aspect of the present invention is a pharmaceutical composition comprising:
a) a therapeutic effective amount of at least one nodal/activin pathway inhibitor, selected from a nodal/activin inhibitor, an ALK4/7 receptor inhibitor or a Samd-2/Smad-4 inhibitor;
b) a therapeutic effective amount of at least one hedgehog pathway inhibitor, selected from a smoothened inhibitor or a Gli inhibitor; and
c) a therapeutic effective amount of at least one chemotherapeutic agent, selected from a nucleoside analog or an antimetabolic agent.

A preferred embodiment of the first aspect of the present invention is a pharmaceutical composition comprising:
a) a therapeutic effective amount of at least one nodal/activin pathway inhibitor, selected from SB431542, SB505124, lefty, follistatin, cerberus, cripto binding protein, coco-protein, nicalin protein and nomo protein;
b) a therapeutic effective amount of at least one hedgehog pathway inhibitor, selected from CUR199691, CUR61414, cyclopamine, cyclopamine-KAAD, Jervine, Forskolin, SANT-1, Arsenic Trioxide (ATO), SIBI-C1, GDC-0449, IPI-926, Gant61, Gant58, Aktar01 1861, Aktar01 1332, Aktar01 1861 and Aktar30337; and
c) a therapeutic effective amount of at least one chemotherapeutic agent, selected from gemcitabine, capecitabine, 5-FU, methotrexate, oxaliplatin, cisplatin, carboplatin, cyclophosphamide, doxorubicine, folic acid, retinoic acid, irinotecan, topotecan, leucovorin, Abraxane, Erlotinib, paclitaxel, docetaxel, vincristine and its derivates, etoposide, teniposide, azathioprine, 6-mercaptopurine.

A preferred embodiment of the first aspect of the present invention is a pharmaceutical composition comprising:
a) a therapeutic effective amount of at least one nodal/activin pathway inhibitor, selected from SB431542 and SB505124;
b) a therapeutic effective amount of at least one hedgehog pathway inhibitor, selected from CUR199691 and CUR61414; and
c) a therapeutic effective amount of at least one chemotherapeutic agent, selected from gemcitabine, cisplatin and paclitaxel.

In a preferred embodiment of the first aspect of the invention, the triple combination comprises SB431542, CUR199691 and gemcitabine.

In a preferred embodiment of the first aspect of the invention, the triple combination consists of SB431542, CUR199691 and gemcitabine.

In a preferred embodiment of the first aspect of the invention, the composition of the invention further comprises a pharmaceutically acceptable excipient.

In a preferred embodiment of the first aspect of the invention, the composition of the invention further comprises at least an additional active ingredient. Preferably, the additional therapeutic agent is an adjuvant.

A second aspect of the present invention refers to a kit of parts comprising:
a) therapeutic effective amount of at least one nodal/activin pathway inhibitor,
b) a therapeutic effective amount of at least one hedgehog pathway inhibitor, and
c) a therapeutic effective amount of at least one chemotherapeutic agent or a pharmaceutically acceptable salt thereof.
wherein the components are provided in a form which is suitable for sequential, separate and/or simultaneous administration.

A third aspect of the present invention refers to the use of the pharmaceutical composition of the first aspect of the inventions or the kit of parts of the second aspect of the invention, as a medicament. In a preferred embodiment of the third aspect of the present invention, the medicament is used in the treatment of cancer. Preferably, the cancer is an epithelial cancer.

In a preferred embodiment of the third aspect of the present invention, the epithelial cancer is pancreatic cancer, colorectal cancer, breast cancer, lung cancer, gastric cancer, liver cancer, biliary cancer, bladder cancer, prostate cancer, thyroid cancer, head and neck cancer, cervical cancer, ovarian cancer, skin cancer, or renal cancer. Preferably, the epithelial cancer is pancreatic cancer.

In a preferred embodiment of the third aspect of the present invention, the administration is oral. The composition of the invention or the medicament comprising it, may be administered in unit dosage form, intestinally, enterally, parenterally or topically, orally, subcutaneously, intranasally, by inhalation, by oral absorption, intravenously, intramuscularly, percutaneously, intraperitoneally, rectally, intravaginally, transdermally, sublingually, buccally, orally transmucosally. Administrative dosage forms may include the following: tablets, capsules, dragees, lozenges, patches, pastilles, gels, pastes, drops, aerosols, pills, powders, liquors, suspensions, emulsions, granules, ointments, creams, suppositories, freeze-dried injections, injectable compositions, in food supplements, nutritional and food bars, syrups, drinks, liquids, cordials etc, which could be regular preparation, delayed-released preparation, controlled-released preparation and various micro-granule delivery system, in food supplements, nutritional and food bars, syrups, drinks, liquids, cordials. In case of tablet, various carriers known in the art may be used, e.g. diluent and resorbent such as starch, dextrin, calcium sulfate, kaolin, microcrystalline cellulose, aluminium silicate, etc; wetting agent and adhesives such as water, glycerin, polyethylene glycol, ethanol, propanol, starch mucilage, dextrin, syrup, honey, glucose solution, acacia, gelatin, carboxymethylcellulose sodium, shellac, methylcellulose, potassium phosphate, polyvinylpyrrolidone, etc; disintegrating agent, such as dried starch, alginate, agar powder, laminaran, sodium bicarbonate and citric acid, calcium carbonate, polyoxyethylene sorbitol aliphatic ester, lauryl sodium sulfate, methylcellulose, ethylcellulose, lactose, sucrose, maltose, mannitol, fructose, various disaccharides and polysaccharides etc; disintegration inhibiting agent, such as sucrose, tristearin, cacao butter, hydrogenated oil, etc; absorption accelerator, such as quaternary ammonium salt, lauryl sodium sulfate, etc; lubricant, such as talc, silica, corn starch, stearate, boric acid, fluid wax, polyethylene, etc. The tablet may be further formulated into coated tablet, e.g. sugar-coated tablet, film-coated tablet, enteric-coated tablet, or double-layer tablet and multi-layer tablet. In the case of pill, various carriers known in the art may be used, e.g. diluent and resorbent, such as glucose, lactose, starch, cacao butter, hydrogenated vegetable oil, polyvinylpyrrolidone, kaolin, talc, etc; adhesives, such as acacia, bassora gum, gelatine, ethanol, honey, liquid sugar, rice paste or flour paste, etc; disintegrating agent, such as agar powder, dried starch, alginate, lauryl sodium sulfate, methylcellulose, ethylcellulose. In case of suppository, various carriers known in the art may be used, e.g. polyethylene, lecithin, cacao butter, higher alcohols, esters of higher alcohols, gelatine, semi-synthetic glyceride, etc. In the case of capsule, it may be prepared by mixing said active substance combinations as active ingredient with the above mentioned carriers, followed by placing the mixture into a hard gelatine capsule or soft capsule. Also, said active substance combinations may be applied in the following dosage forms: microcapsules, suspension in an aqueous phase, hard capsule, or injection. In the case of injection, such as liquor, emulsion, freeze-dried injection, and suspension, all the diluents common in the art may be used, e.g. water, ethanol, polyethylene glycol, propylene glycol, oxyethylated isostearyl alcohol, polyoxidated isostearyl alcohol, polyoxyethylene sorbitol aliphatic ester, etc. In addition, in order to obtain isotonic injection, a suitable amount of sodium chloride, glucose or glycerin may be added into the preparation, as well as regular cosolvent, buffer, pH adjusting agent, etc. In addition, colouring agent, antiseptic, perfume, correctives, food sweetening agents or other materials may be added to the pharmaceutical preparation if necessary.

In a preferred embodiment of the third aspect of the present invention, the administration is parenteral.

In a preferred embodiment of the third aspect of the present invention, the administration reduces the tumour progression.

In a preferred embodiment of the third aspect of the present invention, the medicament is used in the treatment of a mammal. Preferably, the mammal is a human.

The composition of the invention or the medicament comprising it, may be in the form of an injectable liquid, or a physiologically acceptable injectable liquid like a physiological saline solution comprising the active substance combination, to be used for intravenous application. Most preferably the composition of the invention is dissolved in 0.9 % sodium chloride in water without further excipients or additives, desirably with a maximum content of 40 mg/ml of the chemotherapeutic agent, preferably gemcitabine. In another preferred embodiment, the composition of the invention or the medicament comprising it is in the form of a dry powder that can be reconstituted with injectable liquid, or with a physiologically acceptable injectable liquid like a physiological saline solution to comprise the active substance combination, to be used for intravenous application. Most preferably the composition of the invention is ready to be dissolved in 0.9 % sodium chloride in water without further excipients or additives, desirably up to a maximum content of 40 mg/ml of the chemotherapeutic agent, preferably gemcitabine.

### EXAMPLES

### Example 1: Pancreatic cancer stem cells (CSC express pluripotency markers.

We have shown that primary pancreatic CSC can be enriched *in vitro* as floating spherical colonies termed spheres (Hermann P et al., 2007 Cell Stem Cell 1, 313-323). These spheres are composed of a small number of cells with stem cell-like properties including the ability to form secondary spheres. Pancreatic CSC can also be enriched in the CD133- expressing cell population when assessed by flow cytometry (Hermann P et al., 2007 Cell Stem Cell 1, 313-323). For the present studies, we used these two methods for studying pancreatic CSC.

A total number of five human pancreatic adenocarcinoma xenografts (A6L, 185, 354, 265 and 198), some of which have been described previously (Jones et al., 2008 Science 321, 1801-1806), as well as three established pancreatic cancer cell lines (L3.6pl, MiaPaCa2, and BxPC3) were used (Table 1). Primary pancreatic cancer tissues were expanded as xenografts. Isolated cells from these xenografts were cultured as adherent cells or spheres at low passage (Fig. 1 A). Cells were phenotyped by flow cytometry for expression of CD133, CD44, CXCR4, and SSEA-4. As previously reported, we observed that spheres are enriched in CD133+ cells as compared to adherent cells. Moreover, sphere cultures also select for CXCR4 and SSEA-4 positive cells, respectively.

Next, the expression of stemness genes (Nanog, Oct3/4, Stat3, Klf4, and Sox2) was analysed by real-time PCR using first and second-passage spheres and was compared to expression levels in monolayer culture at different levels of confluence. Based on these experiments, we selected day 7 of sphere culture versus 70% confluency in monolayer culture as the optimal condition for comparing mostly differentiated pancreatic cancer cells in adherent cultures and spheres enriched for CSC. As shown in Figure 1 B, expression of stemness genes was significantly higher in spheres as compared to adherent cells. Intriguingly, the expression levels observed for pancreatic spheres were comparable to that of human embryonic stem cells (ESC). To further validate mRNA data at the protein level, we investigated Oct3/4 protein expression by western blotting, which showed that Oct3/4 protein expression was markedly increased in spheres as compared to adherent cells (Fig. 1C).

**Table 1: Mutations in components of the TGF-β superfamily pathways in investigated cell lines.**

| Tumour/cell line TGF-β | Pathway | Reference |
|---|---|---|
| 185 | Smad4 proficient | (Jones et al., 2008 Science 321, 1801-1806) |
| A6L | Smad4 proficient | (Jones et al., 2008 Science 321, 1801-1806) |
| JH051 | Smad4 missense mutation | (Jones et al., 2008 Science 321, 1801-1806) |
| 354 | Smad4 proficient | (Jones et al., 2008 Science 321, 1801-1806) |
| 265 | Smad4 proficient | (Jones et al., 2008 Science 321, 1801-1806) |
| 198 | Smad4 proficient | (Jones et al., 2008 Science 321, 1801-1806) |
| L3.6pi | Smad4 proficient | (Kleeff and Korc, 1998 J Biol Chem 273, 7495-7500) |
| MiaPaCa2 | Deletion of TGF-β type II R | (Freeman et al., 1995 J Cell Physiol 165, 155-163) |
| BxPC3 | Smad4 homozygous deletion | (Subramanian et al., 2004 Cancer Res 64, 5200-5211) |

### Example 2: Components of the Nodal/Activin signalling pathway are overexpressed in primary pancreatic CSC.

Next we determined whether the Nodal/Activin pathway is reactivated in pancreatic cancer (stem) cells by assessing mRNA expression for its components, namely Nodal, Cripto1, FoxH1, Smad2, Smad4, Gdf1, Activin, and Alk4/7. Real-time PCR results showed that the Nodal/Activin signalling-related genes are significantly overexpressed in 1 st generation spheres as compared to adherent cells (Fig. 2A). Interestingly, the expression further strongly increased in 2nd generation spheres (Fig. 2B). Consistently, western blot analysis demonstrated that Nodal is overexpressed in spheres as compared to adherent cells on the protein level (Fig. 2C). In contrast, normal pancreata did not express any Nodal (Fig. 2E). Most importantly, spheres showed enhanced phosphorylation of Smad2, suggesting that the pathway was activated. Moreover, we found that Nodal and pSmad2 are absent or only weakly expressed in adherent cells, while spheres displayed strong cytoplasmic and membranous Nodal protein expression as well as nuclear pSmad2 translocation. Data in established pancreatic cancer cell lines indicated less pronounced differences between spheres and adherent cells (Fig. 8A-B).

### Example 3: Nodal is overexpressed in CD133+ CSC.

By using magnetic activated cells sorting (MACS), CD133+ cells were isolated from digested fresh human pancreatic tumour xenografts (A6L, 185, 198, and 354). Flow cytometry showed good depletion for CD133+ cells in the CD133- population and revealed enrichment to ~75% in the CD133+ population. Real-time PCR analysis showed increased expression of Nodal signalling-associated genes. Specifically, Nodal, Cripto1, Cripto3, Gdf1 and Alk4 are overexpressed in CD133+ cells as compared to CD133- cells (Fig. 2D). Since Nodal expression has recently been shown to be re-activated in breast cancer tissue, we also investigated the modulation of Nodal and its pathway components in MCF7 breast cancer cell line. Consistent with the data obtained for pancreatic cancer, Nodal, its co-factor Cripto-1, and several pluripotency markers were overexpressed in MCF7-derived spheres as compared to adherent cultures (Fig. 9A-B). These results demonstrate that Nodal expression is strongly associated with the cancer stem cell phenotype of epithelial cancers.

### Example 4: Nodal signalling is functionally active in pancreatic cancer cells.

Nodal and Activin are secreted proteins that exert their function by binding to and joining cell surface types I and II receptors to form a tertiary ligand-receptor complex that leads to the phosphorylation of Smad2/3 as intracellular effector and subsequent regulation of cell functions. To determine whether exogenous rNodal and rActivin are capable of activating this signalling cascade, we serum-starved human primary pancreatic cancer cells (A6L and 185) and treated them with either recombinant protein alone or pretreated them with the Alk4/7 inhibitor SB431542 or the Alk5 inhibitor LY2157299 (Fig. 3A). After 30 min of stimulation, phosphorylation of Smad2 was assessed by western blotting and expression of Nanog, Oct3/4, Klf4, Sox2 and Stat3 was evaluated by real-time PCR. Pretreatment with SB431542 or LY2157299 did not affect the viability of adherent cells. Both rNodal and rActivin, but not rTGF-β, strongly induced phosphorylation of Smad2 in adherent cells and spheres (Fig. 3B), while simultaneous treatment with SB431542 almost reversed this enhanced phosphorylation of Smad2. These results suggest that Nodal and Activin are able to activate the signalling by modulation of Smad2 phosphorylation. Moreover, real-time PCR data showed that inhibition of Nodal/Activin signalling decreased the expression of the stemness genes Klf4, Nanog, Sox2, Oct3/4 and STAT3 and some of the Nodal-signalling associated genes (Fig. 10A-B).

### Example 5: Nodal enhances clonogenicity and sphere formation.

To characterize the biological effects of Nodal/Activin on human pancreatic cancer cells, we first examined whether Nodal is capable of enhancing colony formation and self-renewal capacity, respectively, of pancreatic cancer cells. In colony formation assays we observed that addition of recombinant Nodal (rNodal) resulted in more and larger colonies as compared to control (colony size >20µm; 185 cells: 121 ±4 versus 24 ±11; A6L cells: 155±31 versus 26±3). To further characterize the role of Nodal/Activin in regulating CSC self-renewal, we performed sphere formation assays. Single cells in suspension were treated with rNodal, rActivin, rTGF-β, rLefty (endogenous direct inhibitor of Nodal), rFollistatin (endogenous direct inhibitor of Activin), the Alk4/7 inhibitor SB431542 and the Alk5 inhibitor LY2157299. After 7 days, treatment with rNodal increased the number of spheres as compared to control cells. Stimulation with rActivin resulted in increased sphere formation in A6L only. The rather moderate stimulatory effects of rNodal and rActivin are most likely related to already strong Nodal expression in sphere-forming cells as treatment with the Nodal specific inhibitor rLefty (60ng/ml) or the Alk4/7 inhibitor SB431542 dose-dependently (10, 20, and 40µM) blocked sphere formation as compared to control (Fig. 4A). These data further corroborate the crucial importance of the Alk4/7 pathway in the self-renewal capacity of pancreatic CSC. In contrast, the ALK5 inhibitor LY2157299 (10, 20, and 40µM), preferentially blocking TGF-β signalling, did not display any effects on sphere forming capacity (Fig. 4A). Consistently, rNodal and rActivin both enhanced expression of the pluripotency-associated transcription factor Nanog as evidenced by increase activity of the Nanog-RFP reporter (Fig. 4B).

### Example 6: Nodal/Activin stimulate pancreatic cancer cell invasiveness.

Next, we investigated whether Nodal and/or Activin are functionally relevant for cell migration and/or invasion of pancreatic cancer cells. A modified Boyden chamber assay was used to quantitatively evaluate pancreatic cancer cell invasion. As shown in Figure 4C, the percentage of migrated cells increased significantly after stimulation with rNodal and, to lesser extent with rActivin. As opposed to the data for sphere formation, rTGF-β also enhanced invasiveness of CSC. To explore whether Alk4/7-dependent pathways are mediating the effects of Nodal and Activin on invasion, pancreatic cancer cells were pretreated with the Alk4/7 inhibitors SB431542 and SB505124 (both at 20µM). Both inhibitors significantly attenuated the effects of Nodal and Activin on cell invasiveness. Consistently, the Alk5 inhibitor LY2157299 abrogated the enhanced invasiveness of CSC treated with rTGF-β. These data show that both the Nodal/Activin pathway as well as the rTGF-β pathway are capable of enhancing pancreatic cancer stem cell invasiveness.

### Example 7: Knockdown of Nodal reduces self-renewal capacity of pancreatic CSC.

To further validate the role of Nodal signalling on *in vitro* tumorigenicity, we performed gene-silencing experiments using shRNAs that target nonoverlapping Nodal mRNA sequences. To minimize effects of clone-to-clone variation, we isolated independent clones for each shRNA, demonstrating a silencing efficiency in the range from 65-75% (Fig. 5A). These clones showed reduced sphere formation capacity as compared to mock (ShScramble) (Fig. 5B). Interestingly, Nodal downregulation not only decreased sphere numbers, but also the size of the few forming spheres suggesting limited proliferative capacity of cells knocked down for Nodal. Ki67 staining and population-doubling curves verified that adherent cells with Nodal knockdown showed no change in their proliferative capacity (Fig. 5C). The defining characteristic of CSC is their ability to form tumours *in vivo*. Knockdown of Nodal resulted in significantly reduced in vivo tumorigenicity (Fig. 5D), which was accompanied by also reduced Activin mRNA levels.

### Example 8: Nodal/Activin signalling is essential for CSC function.

Our data demonstrate the crucial importance of the Nodal/Activin-Alk4/7 signalling cascade for the self-renewal capacity of pancreatic CSC. In contrast, neither the Alk5 inhibitor LY2157299 nor the TGF-β receptor II neutralizing antibodies resulted in significant changes of sphere forming capacity, suggesting that the TGF-β signalling is not relevant for the self-renewal capacity of CSC (Fig. 4A). Next, we carried out specific genetic targeting of Alk4 and Alk5 utilizing lentiviral delivery of specific shRNA. Knockdown of Alk4, validated by reduced surface expression of Alk4 as assessed by flow cytometry, resulted in a significant reduction of sphere formation capacity and, most importantly, *in vivo* tumorigenicity (Fig. 15A-C). In contrast, knockdown of Alk5, also validated by reduced surface expression of Alk5, neither affected sphere formation capacity nor resulted in reduced *in vivo* tumorigenicity (Fig. 16A-C). Next, knockdown of Smad4, a crucial component of the canonical Alk4/7 signalling cascade, also resulted in a significant reduction of sphere formation capacity (Fig. 11 A-D). Investigating for putative *in vivo* sources for paracrine Nodal/Activin stimulation that could overcome the knockdown of Nodal, we found strong expression of Nodal and Activin in human pancreatic stellate cells (PSC) (Fig. 17A). Sphere formation and invasion of pancreatic CSC were both significantly enhanced by PSC-conditioned medium (Fig. 17B-C). Together with the findings that knockdown of Nodal did translate into reduced sphere formation *in vitro*, but less strikingly reduced tumorigenicity *in vivo*, while knockdown of Alk4 significantly reduced both endpoints, we conclude that *in vivo* CSC are stimulated both in an autocrine and a paracrine fashion by the stroma.

### Example 9: Nodal inhibition eliminates the tumorigenic capacity of pancreatic cancer cells.

Pancreatic CSC are inherently resistant to chemotherapy resulting in enrichment for CSC as evidenced by flow cytometry. This was also associated with an increase in pluripotency genes, both in adherent cells (Fig. 6A) and in spheres (Fig. 6B). Intriguingly, using CD133 expression as readout for CSC content, we observed a virtually complete elimination of CSC by inhibiting the Nodal/Activin pathway. This effect was most consistent in the presence of gemcitabine. These data could be validated in freshly isolated patient pancreatic cancer cells (Fig. 6C).

### Example 10: Nodal inhibition chemosensitises pancreatic cancer stem cells to chemotherapy.

Pancreatic CSC are inherently resistant to chemotherapy resulting in relative enrichment for CSC as evidenced by *in vivo* tumorigenicity (Fig. 12A-B). Tumorigenicity was determined by PET scan on days 32 and macroscopic and microscopic evaluation on day 35. Importantly, only combination therapy was capable of eliminating *in vivo* tumorigenicity. Consistently, using CD133 expression as readout for CSC-content, we observed a virtually complete elimination of CSC by inhibiting the Nodal/Activin pathway. This effect was most consistent in the presence of gemcitabine. These data could be validated in freshly isolated patient pancreatic cancer cells. Mechanistically, we could show that despite a marked decrease in CD133 content following four days of treatment with SB431542 alone, the CD133+ population replenished within 48 hours. If cells were treated with SB431542 and gemcitabine, however, the CD133+ population was irreversibly eliminated. To further elucidate the mechanism of this finding, we performed cell cycle analyses using the BrdU flow kit. Treatment with SB431542 alone did not affect the percentage of CD133+ cells in S phase nor did it increase the percentage of apoptotic CD133+ cells, while the addition of gemcitabine resulted in a 3-fold increase in apoptotic CD133+ cells and virtually complete elimination of cells in S phase. These findings suggest that SB431542 is capable of reversing the chemoresistance of the tumorigenic population, most likely by (reversibly) driving them into a more differentiated state as evidenced by loss of CD133.

### Example 11: In vivo Nodal/Activin pathway inhibition in established pancreatic cancers.

We investigated whether inhibition of Nodal/Activin by SB431542 translates into disease stabilization in pre-established pancreatic cancers. Since only the combination pretreatment with gemcitabine resulted in loss of tumorigenicity *in vivo*, we focused on this treatment regimen. Xenografts were generated by orthotopic implantation of established pancreatic cancer cell lines into immunocompromised mice and treatment was started one week after injection. The detailed experimental setup is depicted in Fig. 7A. The Alk4/7 inhibitor SB431542 was administered for two weeks. Detectable tumour mass was assessed on day 42 by palpation and confirmed by magnetic resonance imaging (Fig. 7B). No tumours were detectable in mice receiving combination therapy so that the study was continued until day 100 to monitor progression/putative relapse of disease. Over time, control animals bore large, life-limiting tumours and succumbed within 40 days after tumour implantation (median survival time 32 days). Gemcitabine alone significantly prolonged survival due to inhibition of tumour growth, but the animals displayed a 100% tumour take rate and their median survival was still severely limited with 54 days. For the combination of SB431542 and gemcitabine, long-term survival was significantly better compared to gemcitabine alone (Fig. 7C).

Next, we investigated the effects of SB431542 in primary human pancreatic cancer tissue xenografts as the ultimate preclinical setting (see study design in Fig. 7D). In contrast to above findings for pancreatic cancer cell lines, the addition of SB431542 to gemcitabine for the treatment of primary pancreatic cancer tissue did not result in a deceleration of tumour growth (Fig. 7E). During long-term follow-up, however, it was only when gemcitabine was already withdrawn that tumour growth eventually started to slow down as compared to tumours treated with gemcitabine alone, while the latter actually reaccelerated in growth. This difference in response to gemcitabine withdrawal resulted in a modest, but significantly reduced tumour burden at 100-day follow-up.

Intriguingly, the triple combination therapy (gemcitabine, SB431542, and CUR) translated into immediate inhibition of tumour progression and eventually translated into long-term stable disease at 100-day follow-up (Fig. 7E-F). Moreover, histological analysis of the remaining tumours on day 100 revealed that remnant tumours in the triple combination group showed abundant evidence for epithelial differentiation with duct-like structures as compared to the gemcitabine group, which showed poor differentiation. Even more importantly, cells isolated from harvested tumours treated with gemcitabine only demonstrated strong sphere forming capacity as opposed to cells isolated from tumours treated with gemcitabine and SB431542, which showed a significant reduction in sphere forming capacity (Fig. 7G, left panel). Most intriguingly, however, cell derived from tumours treated with triple therapy had virtually lost their sphere forming capacity indicative of a complete and irreversible elimination of the CSC pool. Consistent patterns were observed for phenotyping of the cells using flow cytometry. CD133+ or CD133+/CD44+ cells were significantly reduced in the group receiving triple combination therapy (Fig. 7G, right panel). These data demonstrate that the triple therapy is capable of eliminating tumour-promoting pancreatic CSC *in vivo* and were confirmed in a second experiment demonstrating that gemcitabine plus the hedgehog pathway inhibitor did not result in significant long-term disease stabilization, while triple combination did result in tumour regression followed by disease stabilization (Fig. 14).

### Example 12: Methodology:

### Primary human pancreatic cancer cells.

Human pancreatic tumours were obtained with written informed consent from all patients. For *in vitro* studies, tissue fragments were minced, enzymatically digested with collagenase (Stem Cell Technologies, Vancouver, BC) for 90 min at 37°C (Mueller TM et al. Gastroenterology 2009 137(3):1102-13) and after centrifugation for 5 min at 1200 rpm the pellets were resuspended and cultured in RPMI, 10%FBS and 50units/ml pen/strep. Pancreatic cancer spheres were generated by placing the pancreatic cancer cell in suspension (20,000 cells/ml) in serum-free DMEM/F12 media, supplemented with B27 (1:50, Invitrogen), 20ng/ml bFGF and 50units/ml pen/strep. After 7 days of incubation, spheres were typically >75µm big with ~97% CD133high. For serial passaging, 7-day-old spheres were harvested using 40µm cell strainers, dissociated to single cells with trypsin, and then re-grown for 7 days. Cultures were kept no longer than 4 weeks after recovery from frozen stocks.

### Human pancreatic cancer cell lines.

The human pancreatic cancer cell lines L3.6pl, xPC3 and MiaPaca2 were maintained as previously described (Hermann P et al., 2007. Cell Stem Cell 1:313-323).

### In vivo treatment of established pancreatic cancers.

Single-cell suspensions were either orthotopically implanted into the pancreas of female NMRI nu/nu mice (Janvier, Le Genest-Saint-Isle, France) or 2mm3 pieces of primary, *in vivo* expanded pancreatic cancer tissue were subcutaneously implanted, and mice were randomized to the respective treatment groups. Size and weight of the pancreatic tumours were monitored. Gemcitabine was administered twice a week (125 mg/kg i.p.). SB431542 was used at 25 mg/kg and CUR199691 at 100 mg/kg, both by oral gavages twice daily for three weeks.

### Cytometry.

To identify pancreatic CSC, the following antibodies were used: anti-CD133/1-APC or PE; CD133/2-APC (all Miltenyi, Bergisch-Gladbach, Germany) was used for purity testing after MACSorting; EpCAM-FITC, CD44-PE or appropriate isotype-matched control antibodies (Beckton Dickinson, Heidelberg, Germany). Propidium iodide or 7-AAD was used for exclusion of dead cells (eBiosciences, San Diego, CA). Samples were analysed by flow cytometry using a FACS Canto II (BD) and data were analysed with FlowJo 9.2 software (Ashland, Oregon).

### Immunofluorescence.

Primary pancreatic cancer cells and spheres derived were seeded in 96-well dishes (Corning Incorporated, One Riverfront Plaza, NY) and incubated at 37°C for 3h. Cells were washed with cold PBS and then fixed with pre-chilled 4% PFA for 20 min at room temperature. After blocking with 1% bovine serum albumin in PBS-Triton 0.1%, cells were incubated with primary antibodies: Nodal (ab556676; Abcam, Inc.), pSmad2 (3108; Cell Signalling Technology, Inc.) and EpCAM (BD) overnight at 4°C in the dark. Then cells were washed three times with PBS-Triton 0.1% followed by incubation with AlexaFluor-conjugated secondary antibodies against mouse or rabbit (Invitrogen) at room temperature for 1 h in the dark. The cells were mounted in Vectashield containing DAPI (Vector Laboratories, Burlingame, CA) and analysed using an SP5 confocal microscope (Leica, Heidelberg, Germany).

### Western Blot Analysis.

PVDF membranes containing electrophoretically separated proteins from human primary pancreatic cancer cells and spheres were probed with mouse antibodies against Nodal (ab556676; Abcam, Inc.), Smad2 (3103; Cell Signalling Technology, Inc.), GAPDH (ab8245-100; Abcam, Inc.), Smad4 (sc-7966; Santa Cruz Biotechnology, Inc.) and rabbit antibody against pSmad2 (3108; Cell Signalling Technology, Inc.), treated with peroxidase-conjugated goat anti-mouse or anti-rabbit Ig secondary antibody (Sigma), and then visualized by enhanced chemoluminescence (Amersham, Inc.).

### Smad2 phosphorylation assay.

Human primary pancreatic cancer cells were serum-starved for 3h in RPMI (GIBCO). Following starvation, the cells were incubated for 30 min at 37°C with recombinant human Nodal or Activin proteins (300 ng/ml and 100 ng/ml, respectively; R&D) either alone or in the presence of the SB431542 (10 µM, Sigma). Anti-Smad2 and anti-phospho-Smad2 (Ser465/467) Antibodies (Cell Signalling Technology) were used, following the manufacturer's instructions.

### RNA preparation and RT-PCR.

Total RNAs from human primary pancreatic cancer cells and spheres were extracted with TRIzol kit (Life Technologies Inc.) according to the manufacturer's instructions. 1 µg of total RNA was used for cDNA synthesis with SuperScript II reverse transcriptase (Life Technologies Inc.) and random hexamers. Quantitative real-time PCR was performed using SYBR Green PCR master mix (Qiagen), according to the manufacturer's instructions.

The list of utilized primers is in Table 2:

| Gene | Primer sense | Primer antisense |
|---|---|---|
| Nanog | tgaacctcagctacaaacaggtg (SEQ ID NO:1) | aactgcatgcaggactgcagag (SEQ ID NO:2) |
| Oct3/4 | cttgctgcagaagtgggtggaggaa (SEQ ID NO:3) | ctgcagtgtgggtttcgggca (SEQ ID NO:4) |
| Stat3 | aaaactctcacggacgagga (SEQ ID NO:5) | gctgcaactcctccagtttc (SEQ ID NO:6) |
| Klf4 | acccacacaggtgagaaacc (SEQ ID NO:7) | atgtgtaaggcgaggtggtc (SEQ ID NO:8) |
| Sox2 | agaaccccaagatgcacaac (SEQ ID NO:9) | cggggccggtatttataatc (SEQ ID NO:10) |
| Nodal | agcatggttttggaggtgac (SEQ ID NO:11) | cctgcgagaggttggagtag (SEQ ID NO:12) |
| Cripto-1 | tccttctacggacggaactg (SEQ ID NO:13) | atcacagccgggtagaaatg (SEQ ID NO:14) |
| FoxH1 | ccccctaagaggaggaagaa (SEQ ID NO:15) | cttccctgaagaaggggaac (SEQ ID NO:16) |
| Smad2 | cgaaatgccacggtagaaat (SEQ ID NO:17) | ccagaagagcagcaaattcc (SEQ ID NO:18) |
| Smad4 | ccatttccaatcatcctgct (SEQ ID NO:19) | acctttgcctatgtgcaacc (SEQ ID NO:20) |
| Gdf1 | actactgccagggtcagtgc (SEQ ID NO:21) | acgttgtcgctgttgtcaaa (SEQ ID NO:22) |
| Activin | aaagcttcatgtgggcaaag (SEQ ID NO:23) | aatctcgaagtgcagcgtct (SEQ ID NO:24) |
| Alk4 | ggagcgtcttgtctttggag (SEQ ID NO:25) | tgcaacaggatcgacttgag (SEQ ID NO:26) |
| Grp78 | gctattgcttatggcctgga (SEQ ID NO:27) | cgctggtcaaagtcttctcc (SEQ ID NO:28) |
| Cripto-3 | gtgatttggatcatggccatttctaaagc (SEQ ID NO:29) | gctgtcatctctgaaggccaggtc (SEQ ID NO:30) |
| Gapdh | caggagcgagatccct (SEQ ID NO:31) | ggtgctaagcagttggt (SEQ ID NO:32) |
| Tgfb1 | aagtggacatcaacgggttc (SEQ ID NO:33) | tgcggaagtcaatgtacagc (SEQ ID NO:34) |

### Invasion and Migration assays.

Invasion assays were performed using modified Boyden chambers filled with Matrigel™ (BioCoat®, BD Biosciences, Heidelberg, Germany). Cells were pre-treated with 10µM SB431542, SB505124 or recombinant human Lefty for 1 h. 500µl of cell suspensions containing 5x104 pre-treated or untreated cells were added to the Matrigel™ coated inserts, and 750µl of serum-free medium with or without 300ng/ml recombinant human Nodal was added to the lower chamber. The assay chambers were incubated for 22h at 37°C. Invaded cells were fixed in 4% PFA and stained with DAPI. The ratios of cells in the lower chamber versus total seeded cells (in per cent) were calculated.

Transwell migration assays were done using the same procedures as above, except for using filters coated filters with type I collagen (100 Ag/cm2). The data are calculated as the mean ± standard deviation of three measurements. HPF= mean of 10 high-power fields.

### shRNA-mediated knockdown of Nodal, Alk4 and Alk5.

As lentiviral shuttle backbone we used a pLVX shRNA2 plasmid (Clontech). shRNA constructs were generated by hybridization in solution of HPLC-purified paired oligonucleotides with the recessed restriction sites (Bam HI and Eco RI) added to the sequence for cloning purposes. As control we used pLVX-shRNA expression vectors encoding a scrambled shRNA sequence with no target (*in silico* prediction). The shRNA sequences were selected from the RNAi Consortium website (www.broadinstitute.org/rnai). The inserts of shRNA were annealed from sense and antisense oligonucleotides with the sequences as provided in Table 2. Lentivirus production and titration were carried out as previously described in Torres R et al., 2011 PLoS One. 2011;6(5) and regularly contained 1x10⁷ T.U./mL with a 1:100 T.U./physical particles ratio as quantitated by qPCR. Cells were then transduced with lentiviral stocks diluted to an M.O.I. of 50 in the presence of polybrene (8 µg/mL, Sigma). A6L and 185 cells were seeded at a density of 5x10⁴ cells per 24 multi-well plate and allowed to adhere overnight. The next day, cells were infected with the lentivirus for 6 h. Stably transduced cells were obtained after cell sorting for GFP included in the viral vector (for Alk4, Alk5, Nodal) or using puromycin resistance (Smad4).

For the knockdown of gene expressions, A6L and 185 cells were seeded at 5×10⁵ cells per 6-cm dish and allowed to adhere overnight. The next day, cells were transfected with a Nodal specific short hairpin RNA plasmid (shNodal, TRCN0000058699) or the PLKO.1 vector control using Lipofectamine 2000 (Invitrogen). Stably transfected clones were obtained after 21 days of puromycin selection (2.5 µg/ml). All shRNA plasmids and the control vector were purchased from OpenBiosystem.

### Magnetic Resonance imaging (MRI).

Mice were analysed with a 3-Tesla MRI system (Magnetom Tim Trio, Siemens, Erlangen, Germany) using a dedicated small animal coil and T2-weighted scanning.

### Statistical analyses.

Results for continuous variables are presented as means ± standard error of the mean (SEM) unless stated otherwise. Treatment groups were compared with the independent samples t test. Pair-wise multiple comparisons were performed with the one-way ANOVA (two-sided) with Bonferroni adjustment. P values <0.05 were considered statistically significant. All analyses were performed using SPSS 17.0 (SPSS Inc., Chicago, Illinois).

## Claims

1. A pharmaceutical composition comprising a triple combination of:
a) a therapeutic effective amount of at least one nodal/activin pathway inhibitor,
b) a therapeutic effective amount of at least one hedgehog pathway inhibitor, and
c) a therapeutic effective amount of at least one chemotherapeutic agent or a pharmaceutically acceptable salt thereof.

2. The pharmaceutical composition according to claim 1, wherein at least one nodal/activin pathway inhibitor is selected from the list comprising: SB431542, SB505124, lefty, follistatin, cerberus, cripto binding protein, coco-protein, nicalin protein and nomo protein.

3. The pharmaceutical composition according to any of claims 1 or 2, wherein the nodal/activin pathway inhibitor is SB431542.

4. The pharmaceutical composition according to any of claims 1 to 3, wherein at least one hedgehog pathway inhibitor is selected from the list comprising: CUR199691, CUR 61414, cyclopamine, cyclopamine-KAAD, Jervine, Forskolin, SANT-1, Arsenic Trioxide (ATO), SIBI-C1, GDC-0449, IPI-926, Gant61, Gant58, Aktar01 1861, Aktar01 1332, Aktar01 1861 and Aktar30337.

5. The pharmaceutical composition according to any of claims 1 to 4, wherein the hedgehog pathway inhibitor is CUR199691.

6. The pharmaceutical composition according to any of claims 1 to 5, wherein at least one chemotherapeutic agent is selected from the list comprising: gemcitabine, capecitabine, 5-FU, methotrexate, oxaliplatin, cisplatin, carboplatin, cyclophosphamide, doxorubicine, folic acid, retinoic acid, irinotecan, topotecan, leucovorin, Abraxane, erlotinib, paclitaxel, docetaxel, vincristine and its derivates, etoposide, teniposide, azathioprine, and 6-mercaptopurine.

7. The pharmaceutical composition according to any of claims 1 to 6, wherein the chemotherapeutic agent is gemcitabine.

8. The pharmaceutical composition according to any of claims 1 to 7, wherein the triple combination comprises SB431542, CUR199691 and gemcitabine.

9. A kit of parts comprising:
a) therapeutic effective amount of at least one nodal/activin pathway inhibitor,
b) a therapeutic effective amount of at least one hedgehog pathway inhibitor, and
c) a therapeutic effective amount of at least one chemotherapeutic agent or a pharmaceutically acceptable salt thereof.
wherein the components are provided in a form which is suitable for sequential, separate and/or simultaneous administration.

10. The pharmaceutical composition according to any of claims 1 to 8, or the kit of parts according to claim 9, for use as a medicament.

11. The pharmaceutical composition or the kit of parts according to claim 10, for use in the treatment of cancer.

12. The pharmaceutical composition or the kit of parts according to claim 11, wherein the cancer is an epithelial cancer.

13. The pharmaceutical composition or the kit of parts according to claim 12, wherein the epithelial cancer is pancreatic cancer, colorectal cancer, breast cancer, lung cancer, gastric cancer, liver cancer, biliary cancer, bladder cancer, prostate cancer, thyroid cancer, head and neck cancer, cervical cancer, ovarian cancer, skin cancer, or renal cancer.

14. The pharmaceutical composition or the kit of parts according to claim 13 wherein the epithelial cancer is pancreatic cancer.

15. The pharmaceutical composition or the kit of parts according to any of claims 10 to 14 wherein the administration reduces the tumour progression.


SEQUENCE LISTING

<110>  Centro Nacional de Investigaciones Oncológicas CNIO

<120>  Triple combination for the treatment of cancer

<130>  EP1599.11

<160>  34

<170>  PatentIn version 3.5

<210>  1
<211>  23
<212>  DNA
<213>  Homo sapiens

<400>  1
tgaacctcag ctacaaacag gtg                                              23

<210>  2
<211>  22
<212>  DNA
<213>  Homo sapiens

<400>  2
aactgcatgc aggactgcag ag                                               22

<210>  3
<211>  25
<212>  DNA
<213>  Homo sapiens

<400>  3
cttgctgcag aagtgggtgg aggaa                                            25

<210>  4
<211>  21
<212>  DNA
<213>  Homo sapiens

<400>  4
ctgcagtgtg ggtttcgggc a                                                21

<210>  5
<211>  20
<212>  DNA
<213>  Homo sapiens

<400>  5
aaaactctca cggacgagga                                                  20

<210>  6
<211>  20
<212>  DNA
<213>  Homo sapiens

<400>  6
gctgcaactc ctccagtttc                                                  20

<210>  7
<211>  20

<212>  DNA
<213>  Homo sapiens

<400>  7
acccacacag gtgagaaacc                                                    20


<210>  8
<211>  20
<212>  DNA
<213>  Homo sapiens

<400>  8
atgtgtaagg cgaggtggtc                                                    20


<210>  9
<211>  20
<212>  DNA
<213>  Homo sapiens

<400>  9
agaaccccaa gatgcacaac                                                    20


<210>  10
<211>  20
<212>  DNA
<213>  Homo sapiens

<400>  10
cggggccggt atttataatc                                                    20


<210>  11
<211>  20
<212>  DNA
<213>  Homo sapiens

<400>  11
agcatggttt tggaggtgac                                                    20


<210>  12
<211>  20
<212>  DNA
<213>  Homo sapiens

<400>  12
cctgcgagag gttggagtag                                                    20


<210>  13
<211>  20
<212>  DNA
<213>  Homo sapiens

<400>  13
tccttctacg gacggaactg                                                    20


<210>  14
<211>  20
<212>  DNA
<213>  Homo sapiens

<400>  14
atcacagccg ggtagaaatg                                                    20

<210>  15
<211>  20
<212>  DNA
<213>  Homo sapiens

<400>  15
ccccctaaga ggaggaagaa                                                   20


<210>  16
<211>  20
<212>  DNA
<213>  Homo sapiens

<400>  16
cttccctgaa gaaggggaac                                                   20


<210>  17
<211>  20
<212>  DNA
<213>  Homo sapiens

<400>  17
cgaaatgcca cggtagaaat                                                   20


<210>  18
<211>  20
<212>  DNA
<213>  Homo sapiens

<400>  18
ccagaagagc agcaaattcc                                                   20


<210>  19
<211>  20
<212>  DNA
<213>  Homo sapiens

<400>  19
ccatttccaa tcatcctgct                                                   20


<210>  20
<211>  20
<212>  DNA
<213>  Homo sapiens

<400>  20
acctttgcct atgtgcaacc                                                   20


<210>  21
<211>  20
<212>  DNA
<213>  Homo sapiens

<400>  21
actactgcca gggtcagtgc                                                   20


<210>  22
<211>  20
<212>  DNA

<213>  Homo sapiens

<400>  22
acgttgtcgc tgttgtcaaa                                                    20


<210>  23
<211>  20
<212>  DNA
<213>  Homo sapiens

<400>  23
aaagcttcat gtgggcaaag                                                    20


<210>  24
<211>  20
<212>  DNA
<213>  Homo sapiens

<400>  24
aatctcgaag tgcagcgtct                                                    20


<210>  25
<211>  20
<212>  DNA
<213>  Homo sapiens

<400>  25
ggagcgtctt gtctttggag                                                    20


<210>  26
<211>  20
<212>  DNA
<213>  Homo sapiens

<400>  26
tgcaacagga tcgacttgag                                                    20


<210>  27
<211>  20
<212>  DNA
<213>  Homo sapiens

<400>  27
gctattgctt atggcctgga                                                    20


<210>  28
<211>  20
<212>  DNA
<213>  Homo sapiens

<400>  28
cgctggtcaa agtcttctcc                                                    20


<210>  29
<211>  29
<212>  DNA
<213>  Homo sapiens

<400>  29
gtgatttgga tcatggccat ttctaaagc                                          29

<210>  30
<211>  24
<212>  DNA
<213>  Homo sapiens

<400>  30
gctgtcatct ctgaaggcca ggtc                                              24


<210>  31
<211>  16
<212>  DNA
<213>  Homo sapiens

<400>  31
caggagcgag atccct                                                       16


<210>  32
<211>  17
<212>  DNA
<213>  Homo sapiens

<400>  32
ggtgctaagc agttggt                                                      17


<210>  33
<211>  20
<212>  DNA
<213>  Homo sapiens

<400>  33
aagtggacat caacgggttc                                                   20


<210>  34
<211>  20
<212>  DNA
<213>  Homo sapiens

<400>  34
tgcggaagtc aatgtacagc                                                   20